# EUROPEAN PATENT SPECIFICATION

(11) **EP 1 379 535 B1**
(45) Date of publication and mention of the grant of the patent: **13.06.2012**
(21) Application number: 01989844.4
(22) Date of filing: 01.11.2001
(51) Int. Cl.: C07H 21/02

(54) **EXPRESSION VECTORS ABLE TO ELICIT IMPROVED IMMUNE RESPONSE AND METHODS OF USING SAME**
ZUR HERVORRUFUNG EINER VERBESSERTEN IMMUNANTWORT FÄHIGE EXPRESSIONSVEKTOREN UND VERFAHREN ZU DEREN VERWENDUNG
VECTEURS D'EXPRESSION PERMETTANT DE PROVOQUER UNE REPONSE IMMUNITAIRE ET PROCEDES D'UTILISATION DESDITS VECTEURS

(30) Priority: 01.11.2000 US 245113 P
(43) Date of publication of application: 14.01.2004
(73) Proprietor: THE GOVERNMENT OF THE UNITED STATES OF AMERICA, as represented by THE SECRETARY, DEPARTMENT OF HEALTH AND HUMAN SERVICES, Rockville, MD 20852 (US)
(72) Inventor: PAVLAKIS, George, N., Rockville, MD 20850 (US); GRAGEROV, Alexander, Frederick, MD 21701 (US); FELBER, Barbara, K., Rockville, MD 20850 (US)
(74) Representative: Campbell, Patrick John Henry
(86) International application number: PCT/US2001/045624
(87) International publication number: WO 2002/036806

(56) References cited:
- WO-A-00/04926
- WO-A1-99/46392
- BIRAGYN ET AL: "Genetic fusion of chemokines to a self tumor antigen induces protective, T-cell dependent antitumor immunity-" NATURE BIOTECHNOLOGY, NATURE PUBLISHING GROUP, NEW YORK, NY, US, vol. 17, no. 3, 1 March 1999 (1999-03-01), pages 253-258, XP002108131 ISSN: 1087-0156
- KIPPS T ET AL: "Extending genetic vaccines with chemokines." NATURE BIOTECHNOLOGY. MAR 1999, vol. 17, no. 3, March 1999 (1999-03), pages 226-227, XP002400813 ISSN: 1087-0156
- VLEMINCKX K ET AL: "The C-terminal transactivation domain of beta-catenin is necessary and sufficient for signaling by the LEF-1/beta-catenin complex in Xenopus laevis" MECHANISMS OF DEVELOPMENT, ELSEVIER SCIENCE IRELAND LTD, IE, vol. 81, no. 1-2, March 1999 (1999-03), pages 65-74, XP002221397 ISSN: 0925-4773
- TOBERY T W ET AL: "TARGETING OF HIV-1 ANTIGENS FOR RAPID INTRACELLULAR DEGRADATION ENHANCES CYTOTOXIC T LYMPHOCYTE (CTL) RECOGNITION AND THE INDUCTION OF DE NOVEO CTL RESPONSES IN VIVO AFTER IMMUNIZATION" JOURNAL OF EXPERIMENTAL MEDICINE, TOKYO, JP, vol. 185, no. 5, 1997, pages 909-920, XP000671823 ISSN: 0022-1007
- WU Y ET AL: "DEOXYRIBONUCLEIC ACID VACCINES ENCODING ANTIGENS WITH RAPID PROTEASOME-DEPENDENT DEGRADATION ARE HIGHLY EFFICIENT INDUCERS OF CYTOLYTIC T LYMPHOCYTES" JOURNAL OF IMMUNOLOGY, THE WILLIAMS AND WILKINS CO. BALTIMORE, US, vol. 159, 15 December 1997 (1997-12-15), pages 6037-6043, XP002071202 ISSN: 0022-1767
- HERSHKO A ET AL: "THE UBIQUITIN SYSTEM" ANNUAL REVIEW OF BIOCHEMISTRY, PALTO ALTO, CA, US, vol. 67, 1998, pages 425-479, XP008013250 ISSN: 0066-4154
- ROSATI M ET AL: "DNA vaccines expressing different forms of simian immunodeficiency virus antigens decrease viremia upon SIVmac251 challenge" JOURNAL OF VIROLOGY, NEW YORK, US, US, vol. 79, no. 13, July 2005 (2005-07), pages 8480-8492, XP002373828 ISSN: 0022-538X
- TOBERY T.: 'Cutting edge: induction of enhanced CTL-dependent protective immunity in vivo by N-end rule targeting of a model tumor antigen' THE JOURNAL OF IMMUNOLOGY vol. 162, 1999, pages 639 - 642, XP002938798
- BIRAGYN A. ET AL.: 'B-cell malignancies as a model for cancer vaccines: from prototype protein to next generation genetic chemokine fusions' IMMUNOLOGICAL REVIEWS vol. 170, 1999, pages 115 - 126, XP001113113

## Description

### I. TECHNICAL FIELD

Described herein are nucleic acids (such as DNA immunization plasmids), encoding fusion proteins containing a destabilizing amino acid sequence attached to an amino acid sequence of interest, in which the immunogenicity of the amino acid sequence of interest is increased by the presence of the destabilizing amino acid sequence. Also described herein are nucleic acids encoding secreted fusion proteins, such as those containing chemokines or cytokines, and an attached amino acid sequence of interest, in which the immunogenicity of the amino acid sequence of interest is increased as a result of being attached to the secretory sequence. Further described herein are methods of increasing the immunogenicity of the encoded proteins for use as vaccines or in gene therapy.

### II. BACKGROUND

Cellular immune responses against human immunodeficiency virus type 1 (HIV-1) and the related simian immunodeficiency virus (SIV) have been shown to play an important role in controlling HIV-1 and SIV infection and in delaying disease progression. Containment of primary HIV-1 infection in infected individuals correlates with the emergence of virus-specific cytotoxic T-lymphocyte (CTL) responses (1, 2, 3). In chronically infected individuals, a high-frequency CTL response against HIV-1 is also correlated with a low viral load and slow disease progression (4,5). An HIV-1-specific CTL response has also been demonstrated in certain highly exposed seronegative individuals (6, 7, 8). Also, strong HIV-specific proliferative responses, which may be critical for the maintenance of CTL responses, have been identified in long-term nonprogressors (9, 10).

HIV-1 Gag is one of the most conserved viral proteins. Broad, cross-clade CTL responses recognizing conserved epitopes in HIV-1 Gag have been detected in HIV-1 infected people (11, 12), and the development of a safe and effective HIV-1 vaccine may depend on the induction of effective CTL and/or T-helper responses against conserved HIV-1 proteins such as Gag. DNA vaccines have been shown to induce efficient cellular immune responses and protection against a variety of viral, bacterial, and parasitic pathogens in animal models. However, DNA vaccines that could induce potent cellular immune responses against HIV-1 Gag are not yet available.

We have recently demonstrated that by destroying inhibitory sequences in the coding region of HIV-1 gag, we could significantly increase Gag protein expression in primate as well as in mouse cells (13, 14, 15, 16) and dramatically enhance immune repsonse induced by a DNA vaccine (13). Since this new Gag expression vector is Rev/RRE-independent and species-independent, it provides a feasible approach to systematically evaluating the strategies that could lead to the maximum induction of cellular immune responses against HIV Gag molecules in animal models.

Intramuscular (i.m.) administration of a DNA vaccine represents a simple and effective means of inducing both humoral and cellular immune responses (17). There are three potential pathways reponsible for antigen presentation after i.m. injection of DNA. First, muscle cells could take up the DNA, express the encoded protein antigen, and present it to immune cells. Recent data suggest that this pathway is rather unlikely in vivo (18). Second, antigen presenting cells such as dendritic cells attracted to the site of injection may take up the DNA, express the encoded protein, and present it to T and B cells. Third, muscle cells may take up the DNA and express the protein antigen, with the antigen then being transmitted to dendritic cells for presentation. If the second possibility is the case, a protein that is synthesized and degraded in the cytoplasm of dendritic cells would be an excellent target for major histocompatibility complex (MHC) class I presentation and induction of CTL responses. Alternatively, if the third scenario were true, a protein synthesized in the muscle cells that could be targeted efficiently to dendritic cells would induce the best CTL response.

To distinguish among these different possibilities, three different forms of HIV-1 Gag DNA vaccine vectors were constructed and compared for the induction of immune responses. These different forms of Gag included (i) a standard Gag (St-Gag) (also called "WT" gag herein) that assembles into particles, which are efficiently released from cells and become surrounded by host-cell-derived lipid membrane acquired during virus budding; (ii) a cytoplasmic form of Gag (Cy-Gag) that fails to target the plasma membrane and therefore remains in the cytoplasm; and (iii) a secreted form of Gag (Sc-Gag) that is synthesized on the cytoplasmic face of the rough endoplasmic reticulum (ER), transported through the ER and Golgi apparatus, and released as a secreted protein (i.e., not surrounded by a lipid membrane) (19). (Mutant Gag proteins that are not targeted efficiently to the plasma membrane and remain primarily in the cytoplasm were created by destroying the myristylation signal of HIV-1 Gag. Sc-Gag molecules were created by the addition of the t-PA signal peptide sequence to the N terminus of the HIV-1 Gag molecule. This sequence provides a signal for translocation of the secreted protein into the lumen of the ER, for transport through the ER and Golgi apparatus, and for release in the form of Sc-Gag molecules.) (19).

In the study described above, the question of whether targeting HIV-1 Gag to various subcellular compartments could influence the induction of immune responses in DNA-immunized mice was addressed. The results demonstrated that targeting the HIV-1 Gag molecules to different subcellular compartments does indeed influence both the humoral and cellular immune responses that are elicited by i.m. DNA vaccination. Specifically, when these forms of Gag were administered to mice as a DNA vaccine, it was found that the DNA vector encoding the Sc-Gag generated better primary CTL and T-helper responses than did the DNA vector encoding Cy-Gag. Furthermore, the DNA vector encoding the Sc-Gag also generated a higher level of secondary CTL responses than did the DNA vector encoding Cy-Gag after DNA priming and recombinant vaccinia virus-Gag infection. Vaccinia virus titers were notably reduced in the ovaries of mice immunized with Gag DNA vaccine more than 125 days before infection, as compared to the titer in mice that received only the control DNA vector. These data indicated that CD8⁺ T-cell memory elicited by DNA vaccination is functionally relevant and provides protective immunity in this system. The DNA vector encoding the Sc-Gag provided better protection against recombinant vaccinia virus-Gag than did the DNA vector encoding Cy-Gag (19).

Another study has shown that altering the cellular location of glycoprotein D (gD) from bovine herpesvirus 1 by DNA vaccine modulates humoral immune response. Although both the secreted and cytosolic forms of gD induced an IgG2a antibody response, the secreted from of gD induced a stronger IgG1 response than IgG2a response (23). Similar results for Sc-Gag and Cy-Gag were observed in the study described above. On the other hand, St-Gag (also called "WT" gag herein), which is competent for forming virus-like particles, induced a predominantly IgG2a antibody repsonse. This latter data is consistent with the idea that location of antigens after DNA immunization could influence the type and potency of humoral immune responses.

Although DNA vaccines alone have been shown to protect against pathogenic challenges in small animals (24), their performance in primates has been generally disappointing. DNA vaccines, even with repeated boosting, induce only moderate immune responses when compared to live-attenuated virus or recombinant virus vaccines. However, recent studies have demonstrated that heterologuous priming-boosting immunization regimens using DNA plus recombinant modified vaccinia virus Ankara vectors can induce strong cellular immune responses and protection against malaria in mice (25), (26) and SIVmac (27), (28) in monkey models. Although T-cell immune responses induced by DNA immunization are moderate, they are highly focused upon a few specific epitopes, because of the small number of other epitopes expressed by this antigen delivery system. A boost with a recombinant vaccinia virus expressing the same antigen presumably stimulates this population of primed memory T cells. Our data showed that pSc-GAG induced higher memory T-cell responses than other Gag expression vectors as measured by *ex vivo* CTL activity, higher number of CD8⁺ IFN-γ-producing cells after stimulation with MHC class I-restricted HIV-1 Gag-specific peptide, and greater protection against recombinant vaccinia virus-Gag infection (19). These Gag expression vectors may be useful for further evaluation of heterologous priming and boosting with DNA plus viral vector in inducing protective cellular immune responses. Similar strategies could be considered for nonhuman primate models where SIV or simian/human immunodeficiency virus challenge can be evaluated.

There have been several reports regarding the use of t-PA signal peptides in DNA vaccines. In the case of HIV-1 Env DNA vaccine (20), replacing the authentic signal peptide of gp160 with that of t-PA was intended to overcome the Rev/RRE requirement for Env protein expression (21). Replacing the signal peptide sequences of mycobacterial proteins with that of t-PA in DNA vectors has been shown to correlate with more protection against tuberculous challenge in mice, although CTL responses were not measured (22). DNA vectors containing fusion of t-PA peptide with *Plasmodium vivax* antigens did not significantly increase antibody production in mice, and cellular immune responses were not evaluated (39). Whether the t-PA signal peptide can enhance the induction of immune responses for cytoplasmic antigens in general by means of a DNA vaccine strategy requires further investigation.

Other reports, concerning potential cancer vaccines, have demonstrated that active immunizations of human patients with idiotypic vaccines elicited antigen-specific CD8⁺ T-cell responses and antitumor effects (29). Several alternative preclinical strategies to develop vaccines have been previously reported, including fusion of tumor idiotype-derived single chain Fv ("scFv") with cytokines and immunogenic peptides such as interleukin ("IL")-2, IL-4 and granulocyte-macrophage colony-stimulating factor ("GM-CSF") (30, 31, 32). These fusions of scFv with cytokines, toxin fragments and viral peptides predominantly elicit a humoral response with undetectable activation of cell mediated immunity (see Table 2 of ref. 33). In a different approach, the model antigen is rendered immunogenic in mice by genetically fusing it to a chemokine moiety (33, 34, 35). Potent anti-tumor immunity was dependent on the generation of specific andi-idiotypic antibodies and both CD4+ and CD8+ T cells. These researchers hypothesize that administration of these vaccines as fusion proteins or naked DNA vaccines may allow efficient targeting of antigen-presenting cells in vivo. They also propose that chemokine fusion may represent a novel, general strategy for formulating clinically relevant antigens, such as existing or newly identified tumor and HIV antigens into vaccines for cancer and AIDS, respectively, which elicit potent CD8⁺ T-cell immunity (33). These researchers further state that with regard to HIV vaccine development, it has been shown that HIV cannot enter human cells unless it first binds to two types of cell-surface receptors: CD4 and chemokine receptors. The two major variantly tropic HIV viruses infect cells via CCR5 or CXCR4 co-receptors. Therefore, they state that one may envisage a chemokine fusion vaccine for HIV that would elicit not only T-cell and humoral responses against HIV, but possibly could interfere with the binding of HIV to the respective chemokine receptor, thus blocking infection. Finally, they also propose that their strategy may be further improved by modifying and mutating the chemokine moiety, or replacing it with the viral chemokine-like genes, which would reduce the risk of generation of autoantibodies against native chemokines.

Another strategy designed to enhance the induction of antigen-specific CTL responses involves targeting vaccine antigens directly into the MHC class I antigen-processing pathway, thereby providing more of the peptide epitopes that trigger the CTL response. A signal that targets proteins for proteasomal degradation is the assembly of a polyubiquitin chain attached to an accessible Lys residue in the target protein. One factor that influences the rate at which polyubiquitination occurs is the identity of the N-terminal residue of the target protein, as certain non-met N-termini target proteins for rapid degradation by the 26S proteasome. Townsend and others have shown that such "N-end rule" targeting of antigens can enhance their processing and presentation by the class I pathway in an *in vitro* setting. (See reference 36).

Proteins with non-Met N termini have been expressed in cells using fusion constructs in which the coding sequence of the target protein is fused in-frame to the C terminus of the coding sequence of ubiquitin. Ubiquitin is normally made in the cell as a polyprotein that is cleaved by ubiquitin hydrolases at the C-terminus of each ubiquitin subunit, giving rise to individual ubiquitin molecules. These same ubiquitin hydrolases will also cleave the ubiquitin target fusion protein at the C terminus of ubiquitin, exposing the N terminus of the target. In a recent study, Tobery and Siliciano generated ubiquitin fusions to HIV-1 nef with either Met or Arg at the N terminus of nef (UbMNef and UbRNef, respectively) (37). In *in vitro* experiments using vaccinia vectors to express UbMNef and UBRNef, it was shown that although both vectors induced expression of comparable amounts of nef, the form of nef with an Arg residue at the N terminus and a much shorter half-life (*t*_{1/2} = 15 min vs 10 h). Furthermore, immunization of mice with a vaccinia vector expressing the rapidly degraded UbRNef resulted in the induction of a more vigorous nef-specific CTL response than did immunization with a vaccinia vector expressing the stable UbMNef. Tobery and Siliciano conclude that augmenting nef-specific CTL responses by targeting the antigen for rapid cytoplasmic degradation represents an attractive strategy for vaccination against HIV (37).

In a more recent study, Tobery and Siliciano used the viral protein (HIV-1 nef) as a model tumor-associated antigen to evaluate the *in vivo* efficacy of the "N-end rule" targeting strategy for enhancing the induction of *de novo* CTL responses in mice. They state that their results suggest that the "N-end rule" targeting strategy can lead to an enhancement in the induction of CTL that is sufficient to confer protection against a lethal dose of antigen-expressing tumor cells (36).

Vleminckxk et al. (Mechanisms of Development, vol. 81, pages 65-74 (1999) disclose that the C-terminal transactivation domain of beta-catenin is necessary and sufficient for signalling by the LEF-1/beta-catenin complex in *Xenopus laevis.* Wu et al. (J. Immunol, vol. 159, pages 6037-6043 (1997)) disclose that deoxyribonucleic acid vaccines encoding antigens with rapid proteasome-dependant degradation are highly efficient inducers of cytolytic T-lymphocytes. Hershko (Annual Review of Biochemistry, vol. 67, pages 425-479 (1998)) describes the ubiquitin system. Rosati (J. Virol, vol 79, pages 8480-8492 (1998)) discloses that DNA vaccines expressing different forms of simian immunodeficiency virus antigens decrease viremia upon SIVmac251 challenge. WO 99 46392 discloses methods and compositions relating to chemokine-tumor antigen fusion proteins as cancer vaccines. Biragyn et al. (Nat. Biotech, vol. 17, pages 253-258 (1999)) discloses that genetic fusion of chemokines to a self tumor antigen induces protective, T-cell dependent anti-tumor immunity.

In sum, to date, DNA vaccines expressing various antigens have been used to elicit immune responses. In many cases this response in polarized or suboptimal for practical vaccination purposes. The present invention demonstrates that combinations of DNA vaccines containing different forms of antigens, as well as administration of the DNA vaccines to different immunization sites, increase the immune response, and hence, are expected to provide practical DNA vaccination procedures.

### III. SUMMARY OF THE INVENTION

The invention provides a composition comprising one or more vectors expressing:
(i) a nucleic acid construct containing nucleotide sequences encoding a fusion protein comprising a destabilizing amino acid sequence that targets protein to the ubiquitin-proteasome degradation pathway covalently attached to an antigen, in which the immunogenicity of the antigen is increased by the presence of the destabilizing amino acid sequence, and
(ii) a nucleic acid construct encoding a secreted fusion protein comprising a MCP-3 chemokine amino acid sequence covalently attached to the antigen, in which the immunogenicity of the antigen is increased by the presence of the MCP-3 chemokine amino acid sequence.

The invention further provides a pharmaceutical composition comprising one or more vectors of the invention and a pharmaceutically acceptable carrier. The invention additionally provides a pharmaceutical composition of the invention for use in inducing antibodies in a mammal wherein nucleic acid constructs expressed by one or more vectors of the invention are present in said composition in an amount which is effective to induce said antibodies in said mammal. The invention also provides a pharmaceutical composition of the invention for use in inducing cytotoxic and/or helper-inducer T lymphocytes in a mammal wherein said nucleic acid constructs expressed by one or more vectors of the invention are present in said composition in an amount which is effective to induce cytotoxic and/or helper-inducer T lymphocytes in said mammal.

The invention further provides a vaccine composition for use in inducing immunity in a mammal against HIV infection comprising a therapeutically effective amount of one or more vectors of the invention where the antigen sequence is an HIV antigen sequence, and a pharmaceutically acceptable carrier.

The invention additionally provides a pharmaceutical composition comprising viral particles containing one or more vectors of the invention. The invention also provides a pharmaceutical composition of the invention for use in stimulating an immune response against an antigen.

The invention further provides a composition of the invention for use in vaccinating a mammal by inducing antibodies in the mammal, wherein the one or more vectors are present in an amount which is effective to induce said antibodies in said mammal. The invention additionally provides a composition of the invention for use in vaccinating a mammal by inducing cytotoxic and/or helper-inducer T lymphocytes in the mammal, wherein the one or more vectors are present in an amount which is effective to induce cytotoxic and/or helper-inducer T lymphocytes in said mammal.

The invention also provides a kit comprising one or more vectors of the invention.

The invention further provides a first vector for use in a method for vaccinating a mammal by inducing antibodies, or cytotoxic and/or helper-inducer lymphocytes, wherein said method further comprises administration of a second vector,
wherein one of the vectors expresses a nucleic acid construct containing nucleotide sequences encoding a fusion protein comprising a destabilizing amino acid sequence that targets protein to the ubiquitin-proteasome degradation pathway covalently attached to an antigen in which the immunogenicity of the antigen is increased by the presence of the destabilizing amino acid sequence; and
the other vector expresses a nucleic acid construct encoding a secreted fusion protein comprising a MCP-3 chemokine amino acid sequence covalently attached to the antigen, in which the immunogenicity of the antigen is increased by the presence of the MCP-3 chemokine amino acid sequence.

Also described herein are nucleic acids (including, but not limited to, DNA immunization plasmids), encoding fusion proteins comprising a destabilizing amino acid sequence covalently attached to a heterologous amino acid sequence of interest, in which the immunogenicity of the amino acid sequence of interest is increased by the presence of the destabilizing amino acid sequence.

Further described herein are nucleic acids encoding secreted fusion proteins comprising a secretory amino acid sequence, such as those containing chemokines or cytokines, covalently attached to a heterologous amino acid sequence of interest, in which the immunogenicity of the amino acid sequence of interest is increased by the presence of the secretory amino acid sequence.

Also described herein are products produced by the nucleic acids, e.g., mRNA, polypeptides, and viral articles well as vectors and vector systems comprising these nucleic acids. Additionally disclosed are host cells comprising these nucleic acids, vectors, vector systems and/or their products.

The present disclosure also relates to compositions comprising these nucleic acids, vectors, vector systems, products and/or host cells, and methods of using these compositions, either alone or in combination, to stimulate an improved immune response.

Additionally described are methods of using the same or different nucleic acids, vectors, vector systems, products and/or host cells, or compositions thereof, in different sites to enhance the immune response.

Further described are uses of these nucleic acids, vectors, vector systems, host cells and/or compositions to produce mRNA, polypeptides, and/or infectious viral particles, and/or to induce antibodies and/or cytotoxic and/or helper T lymphocytes.

The present disclosure also relates to the use of these nucleic acids, vectors, vector systems, products and/or host cells, or compositions thereof, in gene therapy or as vaccines.

For example, described herein is the use of these nucleic acid constructs, vectors, vector systems and or host cells for use in immunotherapy and immunoprophylaxis, e.g., as a vaccine, or in genetic therapy after expression, in mammals, preferably in humans. The nucleic acid constructs can include or be incorporated into lentiviral vectors, vaccinia vectors, adenovirus vectors, herpesvirus vectors or other expression vectors or they may also be directly injected into tissue cells resulting in efficient expression of the encoded protein or protein fragment These constructs may also be used for *in-vivo* or *in-vitro* gene replacement, e.g., by homologous recombination with a target gene in-situ. They may also be used for transfecting cells *ex-vivo.*

### IV. BRIEF DESCRIPTION OF THE DRAWINGS

Figure 1. Proliferative responses (shown as stimulation index, SI) in mice injected with the indicated vectors or combinations. Vectors are as described in the examples.
Figure 2. Proliferative responses (shown as stimulation index, SI) in mice injected two times with the indicated SIV expression plasmids or combinations. Together = injection of 3 DNAs at the same sites; 3 sites = injections of the same DNAs at separate sites. Vectors are as described in the examples.
Figure 3. Antibody response in monkeys. Two animals (#585, 587) were injected 4x with 5 mg intramuscularly ("i.m.") of MCP3p37gag expression vector. Two animals (#626, 628) were given the same DNA mucosally as liposome-DNA preparations. Titers plotted as reciprocal serum dilutions scoring positive in HIV p24 ELIZA tests.
Figure 4. Percent of IFNgamma + cells in CD8 population after in vitro stimulation with a gag peptide pool in macaques after three vaccinations with either WT+MCP3; WT+CATE; WT+MCP3+CATE; WT; or no vaccination ("Naïve"). (Note: WT means wild-type gag, also referred to as Standard gag (St-gag) herein; MCP3 means MCP3-gag fusions; CATE means ß-catenin-gag fusions).
Figure 5. Percent of IFNgamma + cells in CD8 population after in vitro stimulation with an env peptide pool in macaques after three vaccinations with either WT+MCP3; WT+CATE; WT+MCP3+CATE; WT; or no injection ("Naïve"). (Note: WT means wild type env; MCP3 means MCP3-env fusion; CATE means ß-catenin-env fusions).
Figure 6. Schematic diagram of the SIV envelope encoding vector CMVkan/R-R-SIVgp160CTE.
Figure 7. DNA sequence of the SIV envelope encoding vector CMVkan/R-R-SIVgp160CTE containing a mutated SIV env gene.
Figure 8. Nucleotide and amino acid sequence of MCP3-gp160 env (HIV) fusion.
Figure 9. Nucleotide and protein sequence of the beta-catenin-gp160 env (HIV) fusion.

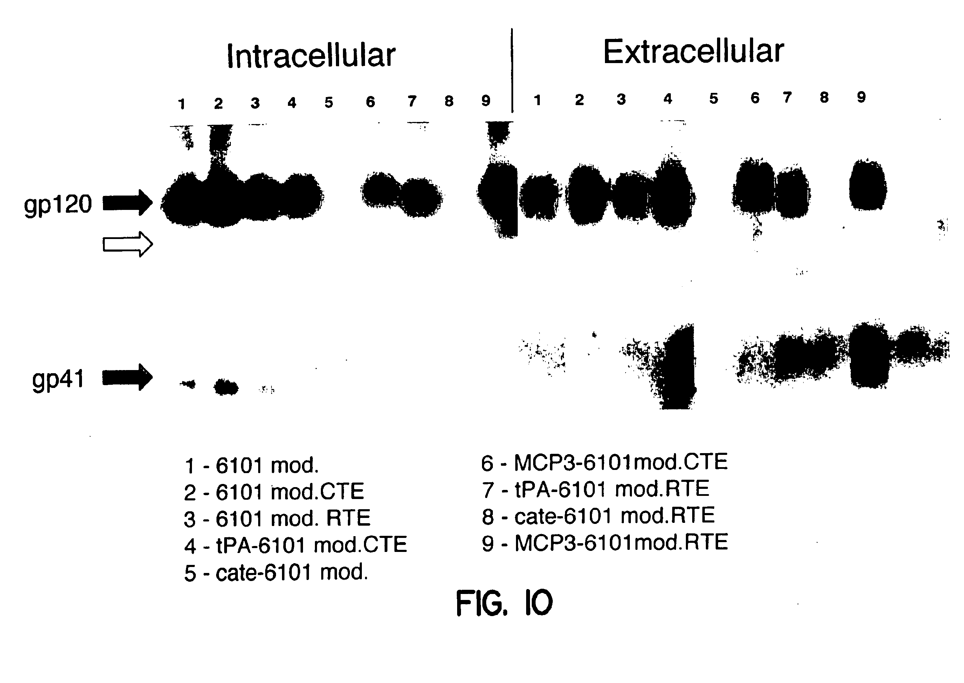

Figure 10. Western blot of HIV env expression vectors. Optimized vectors for wild type sequence of gp160 (lanes 1, 2, 3) or the fusions to MCP-3 (lane 6, 9), tPA leader peptide (lane 4, 7) and beta-catenin (lane 5, 8) are shown. Transfections with purified plasmid DNA were performed in human 293 cells and either cell extracts (intracellular) or cell supernatants (extracellular) were loaded on SDS-acrylamide gels, blotted, and probed with anti-HIV env antibodies. The positions of gp120 and gp41 are shown. Open arrow indicates degradation products detected in lane 5. CTE and RTE indicates respective additional posttranscriptional control elements present in some vectors.

### V. MODES FOR CURRYING OUT THE INVENTION

It is to be understood that both the foregoing general description and the following detailed description are exemplary and explanatory only, and are not restrictive of the invention, as claimed. The accompanying drawings, which are incorporated in and constitute a part of the specification, illustrate an embodiment of the invention and, together with the description, serve to explain the principles of the invention.

Described herein are nucleic acids (including, but not limited to, DNA immunization plasmids), encoding fusion proteins comprising a destabilizing amino acid sequence covalently attached to a heterologous amino acid sequence of interest, in which the immunogenicity of the amino acid sequence of interest is increased by the presence of the destabilizing amino acid sequence.

Also described are nucleic acids encoding secreted fusion proteins comprising a secretory amino acid sequence, such as those containing chemokines or cytokines, covalently attached to a heterologous amino acid sequence of interest, in which the immunogenicity of the amino acid sequence of interest is increased by the presence of the secretory amino acid sequence.

The present disclosure additionally relates to nucleic acids having sequences encoding fusion proteins containing destabilizing amino acid sequences which increase the immunogenicity of an attached amino acid sequence, and to methods of using compositions comprising these nucleic acids, or combinations thereof, to increase the immunogenicity of the encoded protein(s). Further described herein are nucleic acids encoding a fusion protein containing MCP-3 amino acid sequences and HIV gag or env, or SIV gag or env, and additional proteins related to vaccinations against non-tumor associated antigens, such as pathogen antigens. Also described are methods of using different immunization sites to increase the immunogenicity of the encoded protein(s).

One aspect of the present disclosure relates to a nucleic acid construct encoding a fusion protein comprising a destabilization sequence covalently linked to an amino acid sequence containing one or more disease-associated antigen. Preferred destabilization sequences are those which target the fusion protein to the ubiquitin proteosomal degradation pathway. More preferably, the destabilization sequence is present in the amino acid sequences selected from the group consisting of c-Myc aa2-120; Cyclin A aa13-91; Cyclin B aa13-91; IkBa aa20-45; β-Catenin aa19-44; c-Jun aa1-67; and c-Mos aa1-35, and functional fragments thereof.

In one embodiment, the present disclosure relates to nucleic acids comprising sequences which encode polypeptides containing a destabilizing amino acid sequence which increases the immunogenicity of a covalently attached amino acid sequence containing a clinically relevant antigen, such as a disease associated antigen, as compared to its immunogenicity in the absence of the destabilizing amino acid sequence.

In another embodiment, the present disclosure relates to nucleic acids encoding secreted fusion proteins, such as those containing immunostimulatory chemokines, such as MCP-3 or IP-10, or cytokines, such as GM-CSF, IL-4 or IL-2. In a preferred embodiment, the invention relates to fusion proteins containing MCP-3 amino acid sequences and viral antigens such as HIV gag and env or SIV gag or env.

The nucleic acid sequences of the constructs described herein can be synthetic (e.g., synthesized by chemical synthesis), semi-synthetic (e.g., a combination of genomic DNA, cDNA, or PCR amplified DNA and synthetic DNA), or recombinantly produced. The nucleic acid sequences also may optionally not contain introns. The nucleic acid sequence encoding the destabilizing amino acid sequence is preferably linked in frame to the N - terminal of a nucleic acid sequence encoding one or more antigen(s) of interest, or immunogenic epitope(s) thereof. These sequences may optionally be linked by another sequence encoding one or more linker amino acids.

In addition, nucleic acid sequences encoding more than one antigens of interest, may optionally be operably linked in frame or via an internal ribosomal entry site (IRES), e.g., from picornaviral RNA. An IRES will be used in circumstances that one wants to express two proteins (or antigens) from the same promoter. Using an IRES the expression of the two proteins is coordinated. A further polypeptide encoding sequence may also be present under the control of a separate promoter. Such a sequence may encode, for example, a selectable marker, or further antigen(s) of interest. Expression of this sequence may be constitutive; for example, in the case of a selectable marker this may be useful for selecting successfully transfected packaging cells, or packaging cells which are producing particularly high titers of vector particles. Alternatively or additionally, the selectable marker may be useful for selecting cells which have been successfully infected with nucleic acid sequence and have the sequence integrated into their own genome.

The constructs described herein may also encode additional immunostimulation molecules, such as the chemokine MCP-3 exemplified herein, and functional fragments thereof. These immunostimulation molecules may be encoded by nucleic acid sequences as part of the fusion protein expression unit or may be encoded by nucleic acid sequences as part of a separate expression unit. These molecules may also be encoded by sequences present on different nucleic acid constructs, vectors, etc. Immunostimulatory molecules such as cytokines, chemokines or lymphokines are well known in the art. See, e.g., U.S. Patent 6,100,387. See, also, e.g., Biragyn and Kwack (1999) (ref. 34).

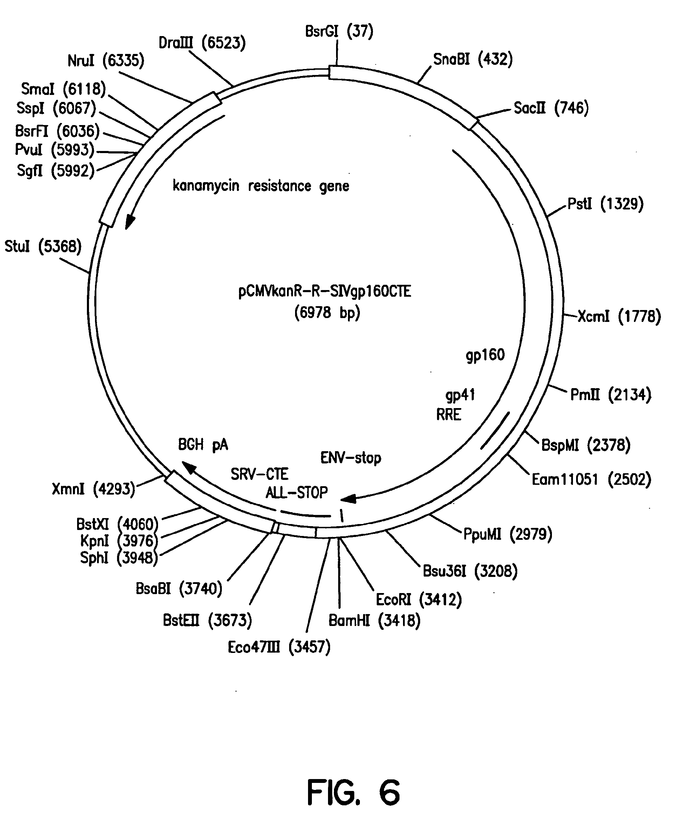

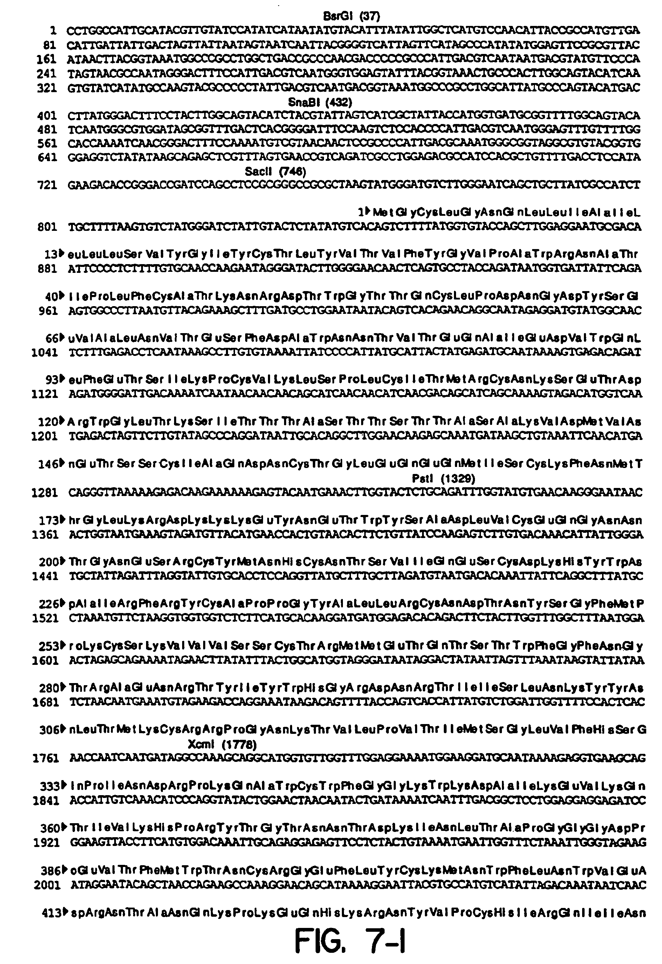

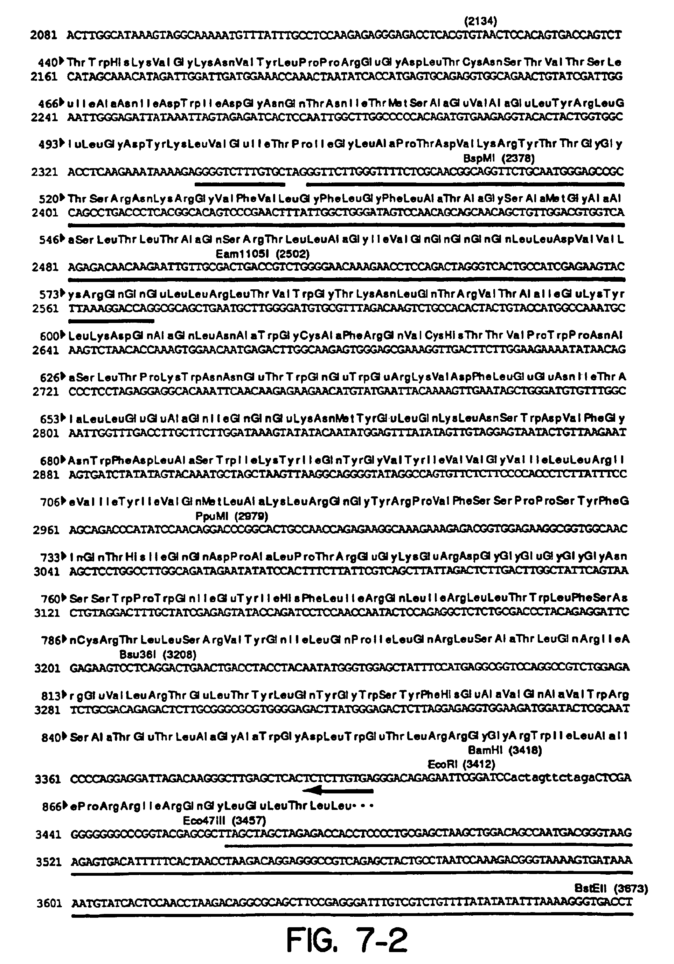

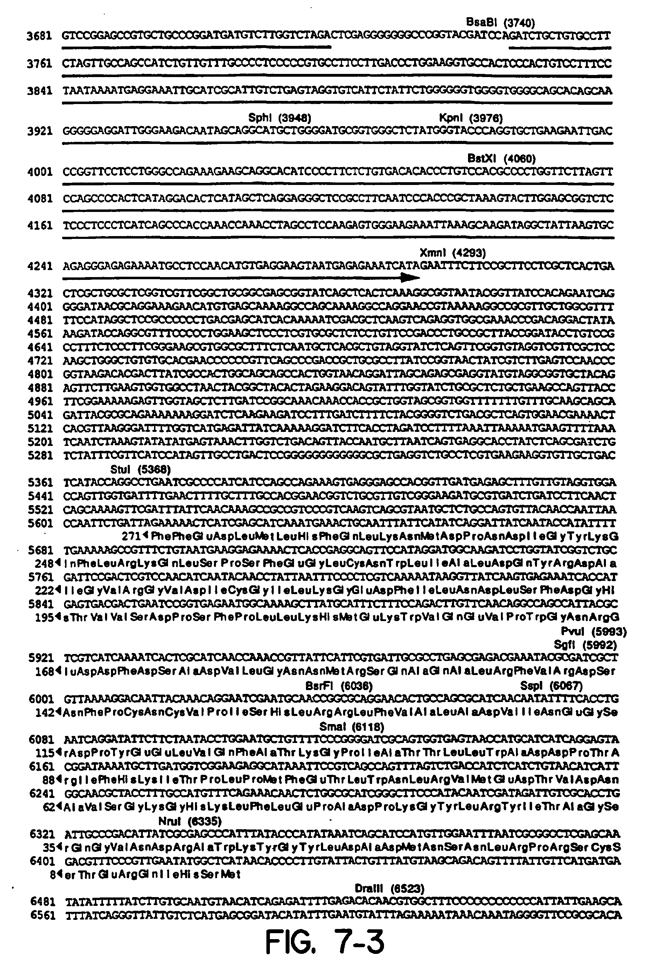

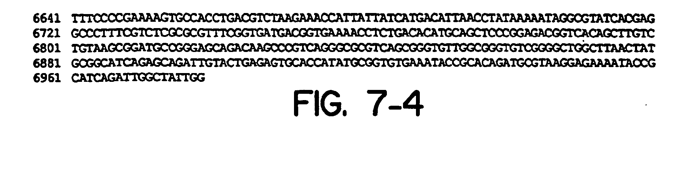

When HIV or SIV antigens are encoded, the nucleic acids may also contain Rev-independent fragments of genes which retain the desired function (e.g., for antigenicity of Gag or Pol, particle formation (Gag) or enzymatic activity (Pol)), or they may also contain Rev-independent variants which have been mutated so that the encoded protein loses a function that is unwanted in certain circumstances. In the latter case, for example, the gene may be modified to encode mutations (at the amino acid level) in the active site of reverse transcriptase or integrase proteins to prevent reverse transcription or integration. Rev-independent fragments of the gag gene and env gene are described in U.S. Patent Nos. 5,972,596 and 5,965,726. See also, PCT/US00/34985 filed December 22, 2000 (published as WO 01/46408 on June 28, 2001) for the gag gene and Figures 6 and 7 herein for the SIV env gene.

The expression of the proteins encoded by these nucleic acid constructs or vectors after transfection into cells may be monitored at both the level of RNA and protein production. RNA levels are quantitated by methods known in the art, e.g., Northern blots, S1 mapping or PCR methods. Protein levels may also be quantitated by methods known in the art, e.g., western blot or ELISA or fluorescent detection methods. A fast non-radioactive ELISA protocol can be used to detect gag protein (DUPONT or COULTER gag antigen capture assay).

Various vectors are known in the art. See, e.g., U.S. Patent 6,100,387. Preferred vectors considered useful in gene therapy and/or as a vaccine vectors, are lentiviral having, depending on the desired circumstances,
a) no round of replication (i.e., a zero replication system)
b) one round of replication, or
c) a fully replicating system

Such vectors are described, e.g., in PCT/US00/34985 filed December 22, 2000 (published as WO 01/46408 on June 28, 2001); and U.S. Serial No. 09/872,733, filed June 1, 2001.

In a preferred embodiment, a HIV- or SIV- based lentiviral system useful here in comprises the following three components:
1) a packaging vector containing nucleic acid sequences encoding the elements necessary for vector packaging such as structural proteins (except for HIV *env*) and the enzymes required to generate vector particles, the packaging vector comprising at least a mutated Rev-independent HIV or SIV *gag*/*pol* gene;
2) a transfer vector containing genetic cis-acting sequences necessary for the vector to infect the target cell and for transfer of the therapeutic or reporter or other gene(s) of interest, the transfer vector comprising the encapsidation signal and the gene(s) of interest or a cloning site for inserting the gene(s) of interest;
   and
3) a vector containing sequences encoding an element necessary for targeting the viral particle to the intended recipient cell, preferably the gene encoding the G glycoprotein of the vesicular stomatis virus (VSV-G) or amphotrophic MuLV or lentiviral *envs.*

In such vectors, when the CMV promoter or other strong, high efficiency, promoter is used instead of the HIV-1 LTR promoter in the packaging vector, high expression of *gag, pol, or gag*/*pol can* be achieved in the total absence of any other viral protein. The exchange of the HIV-1 LTR promoter with other promoters is beneficial in the packaging vector or other vectors if constitutive expression is desirable and also for expression in mammalian cells other than human cells, such as mouse cells, in which the HIV-1 promoter is weak. In certain embodiments, the presence of heterologous promoters will also be desired in the transfer vector and the envelope encoding vector, when such vectors are used.

The antigens of interest, in particular, clinically relevant antigens, are chosen according to the effect sought to be achieved. Preferably, the antigen induces antibodies or helper T-cells or cytotoxic T-cells.

Amino acids, or antigens, of interest useful the nucleic acid constructs of the present disclosure are described, e.g., in U.S. Patent 5,891,432 (see, e.g., Col. 13, In. 20 to Col. 17, In. 67). These antigens include, but are not limited to, disease associated antigens such as tumor-associated antigens, autoimmune disease-associated antigens, infectious disease-associated antigens, viral antigens, parasitic antigens and bacterial antigens. Tumor associated antigens include, but are not limited to, p53 and mutants thereof, Ras and mutants thereof, a Bcr/Abl breakpoint peptide, HER-2/neu, HPV 2, E6, HPV E7, carcinoembryonic antigen, MUC-1, MAGE-1, MAGE-3, BAGE, GAGE-1, GAGE-2, N-acetylglucosaminyltransferase-V, p15, gp100, MART-1/MelanA, tyrosinase, Trip-1, beta-catenin, MUM-1 and CDK-4, N-acetylglucosaminyltransferase-V, p15, gp100, MART-1/MelanA, tyrosinase, TRP-1, beta-catenin, MUM-1 and CDK-4. HIV or SIV antigens include, but are not limited to Gag, Env, Pol, Nef, Vpr, Vpu, Vif Tat and Rev. In a preferred embodiment of the invention, the HIV Gag-Pol-Tat-Rev-Nef or Tat-Rev-Env-Nef antigens are linked together, but are not active as HIV components.

Nucleic acid constructs described herein, as well as vectors, vector systems or viral particles containing such nucleic acid constructs, or the encoded proteins may be used for gene therapy *in vivo* (e.g., parenteral inoculation of high titer vector) or *ex vivo* (e.g., *in vitro* transduction of patient's cells followed by reinfusion into the patient of the transduced cells). These procedures are been already used in different approved gene therapy protocols.

One way of performing gene therapy is to extract cells from a patient, infect the extracted cells with a vector, such as a lentiviral vector, or a viral particle and reintroduce the cells back into the patient. A selectable marker may be used to provide a means for enriching for infected or transduced cells or positively selecting for only those cells which have been infected or transduced, before reintroducing the cells into the patient. This procedure may increase the chances of success of the therapy. Selectable markers may be for instance drug resistance genes, metabolic enzyme genes, or any other selectable markers known in the art. Typical selection genes encode proteins that confer resistance to antibiotics and other toxic substances, e.g., histidinol, puromycin, hygromycin, neomycin, methotrexate, etc., and cell surface markers.

However, it will be evident that for many gene therapy applications of vectors, such as lentiviral vectors, selection for expression of a marker gene may not be possible or necessary. Indeed expression of a selection marker, while convenient for *in vitro* studies, could be deleterious *in vivo* because of the inappropriate induction of cytotoxic T lymphocytes (CTLs) directed against the foreign marker protein. Also, it is possible that for *in vivo* applications, vectors without any internal promoters will be preferable. The presence of internal promoters can affect for example the transduction titres obtainable from a packaging cell line and the stability of the integrated vector. Thus, single transcription unit vectors, which may be bi-cistronic or poly-cistronic, coding for one or two or more therapeutic genes, may be the preferred vector designed for use *in vivo.* See, e.g., WO 98/17816.

Vaccines and pharmaceutical compositions comprising at least one of the nucleic acid sequences, polypeptides, viral particles, vectors vector systems, or transduced or transfected host cells of the present disclosure and a physiologically acceptable carrier are also described herein.

As used herein, the term "transduction" generally refers to the transfer of genetic material into the host via infection, e.g., in this case by the lentiviral vector. The term "transfection" generally refers to the transfer of isolated genetic material into cells via the use of specific transfection agents (e.g., calcium phosphate, DEAE Dextran, lipid formulations, gold particles, and other microparticles) that cross the cytoplasmic membrane and deliver some of the genetic material into the cell nucleus.

### Pharmaceutical Compositions

The pharmaceutical compositions described herein contain a pharmaceutically and/or therapeutically effective amount of at least one nucleic acid construct, polypeptide, vector, vector system, viral particle/virus stock, or host cell (i.e., agents) of the present disclosure. If desired, the nucleic acid constructs, polypeptides, viral particles, vectors, vector systems, viral particle/virus stock, or host cells in described herein can be isolated and/or purified by methods known in the art.

In one embodiment of the invention, the effective amount of an agent of the invention per unit dose is an amount sufficient to cause the detectable expression of the antigen of interest. In another embodiment of the invention, the effective amount of agent per unit dose is an amount sufficient to prevent, treat or protect against deleterious effects (including severity, duration, or extent of symptoms) of the condition being treated. The effective amount of agent per unit dose depends, among other things, on the species of mammal inoculated, the body weight of the mammal and the chosen inoculation regimen, as is well known in the art. The dosage of the therapeutic agents which will be most suitable for prophylaxis or treatment will also vary with the form of administration, the particular agent chosen and the physiological characteristics of the particular patient under treatment. The dose is administered at least once. Subsequent doses may be administered as indicated.

To monitor the response of individuals administered the compositions of the invention, mRNA or protein expression levels may be determined. In many instances it will be sufficient to assess the expression level in serum or plasma obtained from such an individual. Decisions as to whether to administer another dose or to change the amount of the composition administered to the individual may be at least partially based on the expression levels.

The term "unit dose" as it pertains to the inocula refers to physically discrete units suitable as unitary dosages for mammals, each unit containing a predetermined quantity of active material (e.g., nucleic acid, virus stock or host cell) calculated to produce the desired effect in association with the required diluent. The titers of the virus stocks to be administered to a cell or animal will depend on the application and on type of delivery (e.g., *in vivo* or ex *vivo*). The virus stocks can be concentrated using methods such as centrifugation. The titers to be administered *ex vivo* are preferably in the range of 0.001 to 1 infectious unit /cell. Another method of generating viral stocks is to cocultivate stable cell lines expressing the virus with the target cells. This method has been used to achieve better results when using traditional retroviral vectors because the cells can be infected over a longer period of time and they have the chance to be infected with multiple copies of the vector.

For *in vivo* administration of nucleic acid constructs, vectors, vector systems, virus stocks, or cells which have been transduced or transfected *ex vivo,* the dose is to be determined by dose escalation, with the upper dose being limited by the onset of unacceptable adverse effects. Preliminary starting doses may be extrapolated from experiments using lentiviral vectors in animal models, by methods known in the art, or may be extrapolated from comparisons with known retroviral (e.g., adenoviral) doses. Generally, small dosages will be used initially and, if necessary, will be increased by small increments until the optimum effect under the circumstances is reached. Exemplary dosages are within the range of 10⁸ up to approximately 5 x 10¹⁵ particles.

For vaccinations DNA will be administered either IM in PBS as previously described in liposomes, by intradermal inoculation, electro-injection or other methods. As example, 5 mg per dose IM in macaques (DNA at 1mg/ml) injected at several different sites was found to produce a good immune response.

Inocula are typically prepared as a solution in a physiologically acceptable carrier such as saline, phosphate-buffered saline and the like to form an aqueous pharmaceutical composition.

The agents described herein are generally administered with a physiologically acceptable carrier or vehicle therefor. A physiologically acceptable carrier is one that does not cause an adverse physical reaction upon administration and one in which the nucleic acids or other agents of the invention are sufficiently soluble to retain their activity to deliver a pharmaceutically or therapeutically effective amount of the compound. The pharmaceutically or therapeutically effective amount and method of administration of an agent may vary based on the individual patient, the indication being treated and other criteria evident to one of ordinary skill in the art. A nucleic acid construct described herein is preferably present in an amount which is capable of expressing the encoded protein in an amount which is effective to induce antibodies and/or cytotoxic and/or helper-inducer T lymphocytes. A therapeutically effective amount of a nucleic acid is one sufficient to prevent, or attenuate the severity, extent or duration of the deleterious effects of the condition being treated without causing significant adverse side effects. The route(s) of administration useful in a particular application are apparent to one or ordinary skill in the art.

Routes of administration of the agents described herein include, but are not limited to, parenteral, and direct injection into an affected site. Parenteral routes of administration include but are not limited to intravenous, intramuscular, intraperitoneal and subcutaneous. The route of administration of the agents is typically parenteral and is preferably into the bone marrow, into the CSF intramuscular, subcutaneous, intradermal, intraocular, intracranial, intranasal, and the like. See, e.g., WO 99/04026 for examples of formulations and routes of administration.

The present disclosure includes compositions of the agents described above, suitable for parenteral administration including, but not limited to, pharmaceutically acceptable sterile isotonic solutions. Such solutions include, but are not limited to, saline and phosphate buffered saline for nasal, intravenous, intramuscular, intraperitoneal, subcutaneous or direct injection into a joint or other area.

In providing the agents to a recipient mammal, preferably a human, the dosage administered will vary depending upon such factors as the mammal's age, weight, height, sex, general medical condition, previous medical history and the like.

The administration of the pharmaceutical compositions of the invention may be for either "prophylactic" or "therapeutic" purpose. When provided prophylactically, the compositions are provided in advance of any symptom. The prophylactic administration of the composition serves to prevent or ameliorate any subsequent deleterious effects (including severity, duration, or extent of symptoms) of the condition being treated. When provided therapeutically, the composition is provided at (or shortly after) the onset of a symptom of the condition being treated.

For all therapeutic, prophylactic and diagnostic uses, one or more of the agents, as well as antibodies and other necessary reagents and appropriate devices and accessories, may be provided in kit form so as to be readily available and easily used.

Where immunoassays are involved, such kits may contain a solid support, such as a membrane (e.g., nitrocellulose), a bead, sphere, test tube, rod, and so forth, to which a receptor such as an antibody specific for the target molecule will bind. Such kits can also include a second receptor, such as a labeled antibody. Such kits can be used for sandwich assays to detect toxins. Kits for competitive assays are also envisioned.

### VI. INDUSTRIAL APPLICABILITY

The nucleic acids described herein can be expressed in the native host cell or organism or in a different cell or organism. The mutated genes can be introduced into a vector such as a plasmid, cosmid, phage, virus or mini-chromosome and inserted into a host cell or organism by methods well known in the art. In general, the constructs can be utilized in any cell, either eukaryotic or prokaryotic, including mammalian cells (e.g., human (e.g., HeLa), monkey (e.g., Cos), rabbit (e.g., rabbit reticulocytes), rat, hamster (e.g., CHO and baby hamster kidney cells) or mouse cells (e.g., L cells), plant cells, yeast cells, insect cells or bacterial cells (e.g., E. coli). The vectors which can be utilized to clone and/or express nucleic acid sequences described herein are the vectors which are capable of replicating and/or expressing the coding sequences in the host cell in which the coding sequences are desired to be replicated and/or expressed. See, e.g., F. Ausubel et al., Current Protocols in Molecular Biology, Greene Publishing Associates and Wiley-Interscience (1992) and Sambrook et al. (1989) for examples of appropriate vectors for various types of host cells. The native promoters for such coding sequences can be replaced with strong promoters compatible with the host into which the coding sequences are inserted. These promoters may be inducible. The host cells containing these coding sequences can be used to express large amounts of the protein useful in enzyme preparations, pharmaceuticals, diagnostic reagents, vaccines and therapeutics.

The constructs described herein may also be used for in-vivo or in-vitro gene therapy. For example, a construct will produce an mRNA in situ to ultimately increase the amount of polypeptide expressed. Such polypeptides include viral antigens and/or cellular antigens. Such a constructs, and their expression products, are expected to be useful, for example, in the development of a vaccine and/or genetic therapy.

The constructs and/or products made by using constructs encoding antigens of interest could be used, for example, in the production of diagnostic reagents, vaccines and therapies for diseases, such as AIDS and AIDS-related diseases.

For example, vectors expressing high levels of Gag can be used in immunotherapy and immunoprophylaxis, after expression in humans. Such vectors include retroviral vectors and also include direct injection of DNA into muscle cells or other receptive cells, resulting in the efficient expression of gag, using the technology described, for example, in Wolff et al., Science 247:1465-1468 (1990), Wolff et al., Human Molecular Genetics 1(6):363-369 (1992) and Ulmer et al., Science 259:1745-1749 (1993). Further, the gag constructs could be used in transdominant inhibition of HIV expression after the introduction into humans. For this application, for example, appropriate vectors or DNA molecules expressing high levels of p55^{gag} or p37^{gag} would be modified to generate transdominant gag mutants, as described, for example, in Trono et al., Cell 59:113-120 (1989). The vectors would be introduced into humans, resulting in the inhibition of HIV production due to the combined mechanisms of gag transdominant inhibition and of immunostimulation by the produced gag protein. In addition, the gag encoding constructs could be used in the generation of new retroviral vectors based on the expression of lentiviral gag proteins. Lentiviruses have unique characteristics that may allow the targeting and efficient infection of non-dividing cells. Similar applications are expected for vectors expressing high levels of env.

The following examples illustrate certain embodiments of the present invention, but should not be construed as limiting its scope in any way. Certain modifications and variations will be apparent to those skilled in the art from the teachings of the foregoing disclosure and the following examples.

### EXAMPLE 1

### Vectors

DNA vectors expressing antigens of HIV-1 or SIV are used in the examples herein.

Three different types of plasmids encoding forms of HIV Gag exemplified herein are as follows:
1) plasmids expressing full gag (p55) or parts of gag (p37) or gag and protease (p55gagpro). P55 produces gag particles that are partially released from the cell. P37 is partially released from the cell but does not form particles. P55gagpro also produces protease, therefore the gag is processed to form p17, p24, p6 and p7;
2) plasmids expressing the chemokine MCP-3 fused to the N terminus of p55gag. Since MCP-3 is a secreted protein, the produced fusion protein is also secreted from the mammalian cells after the cleavage of the signal peptide; and
3) plasmids expressing fusions of gag to sequences conferring efficient proteasomal degratation.

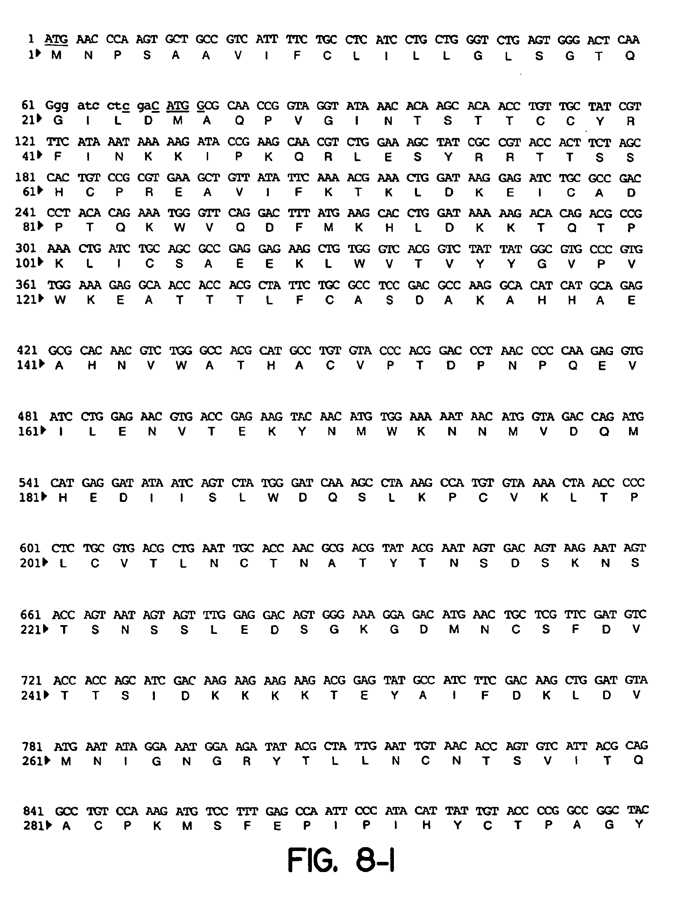

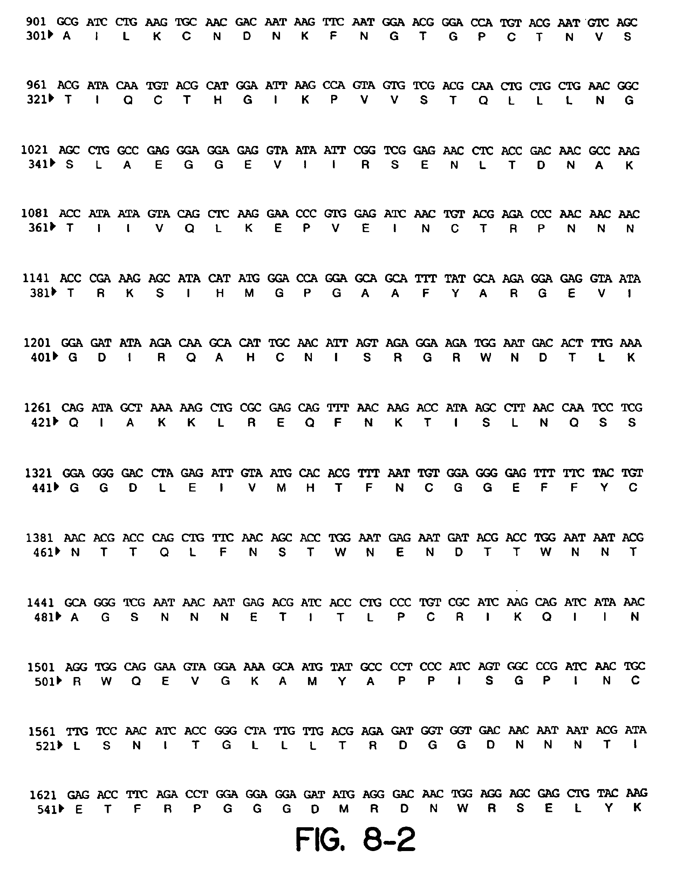

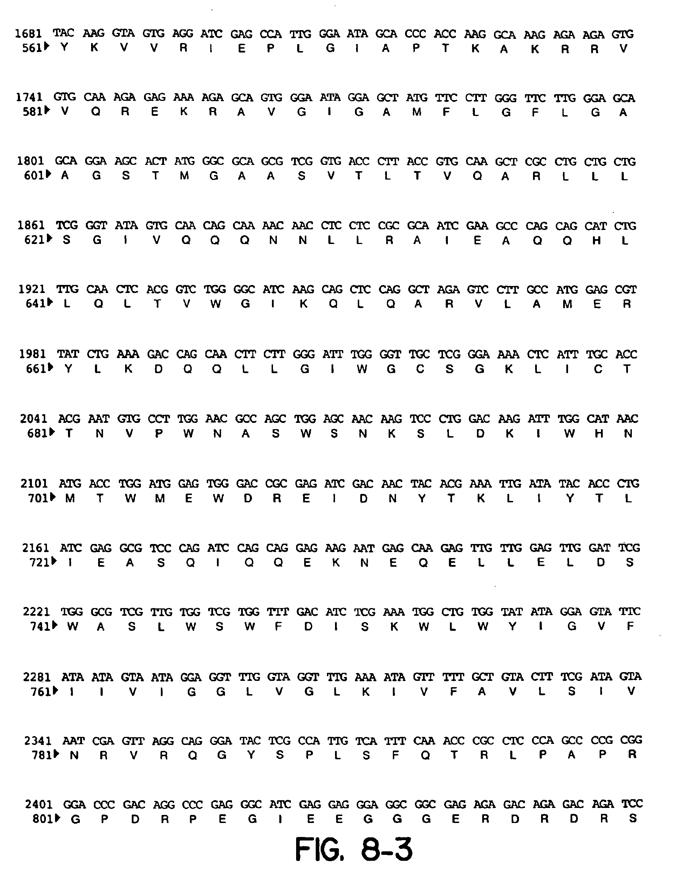

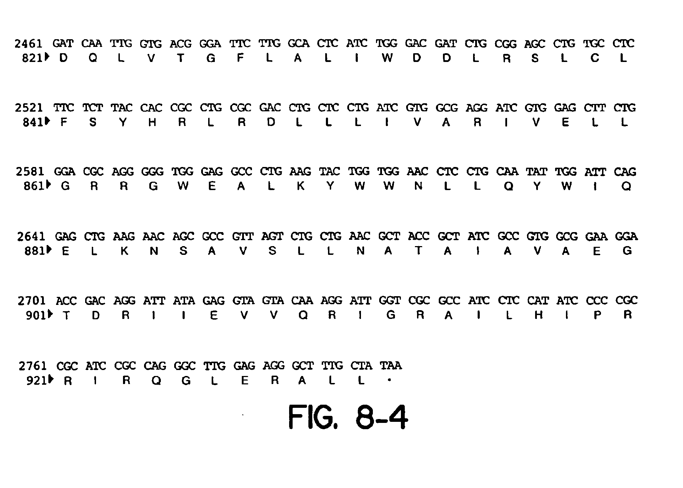

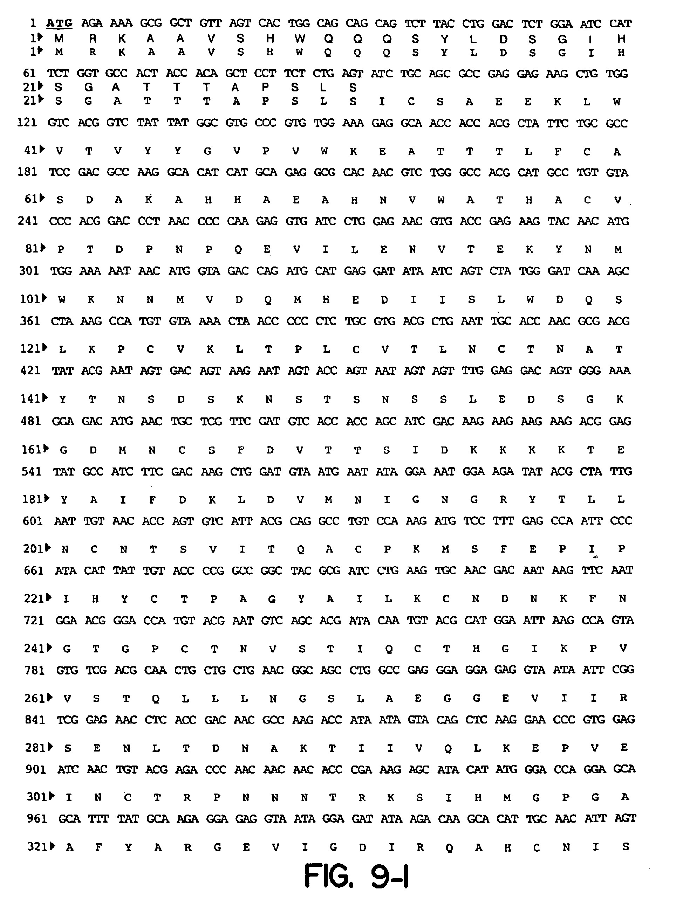

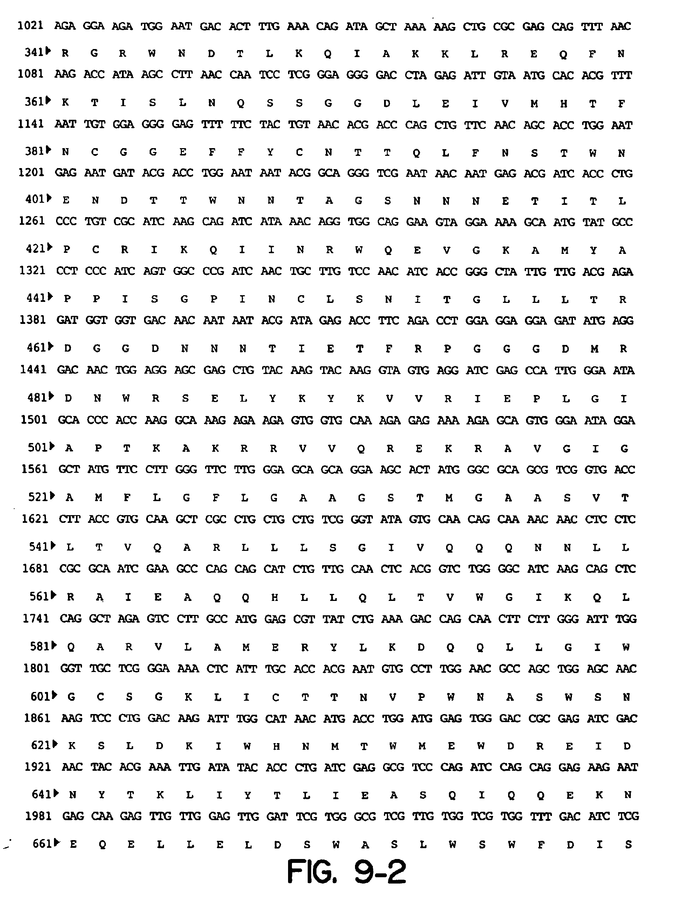

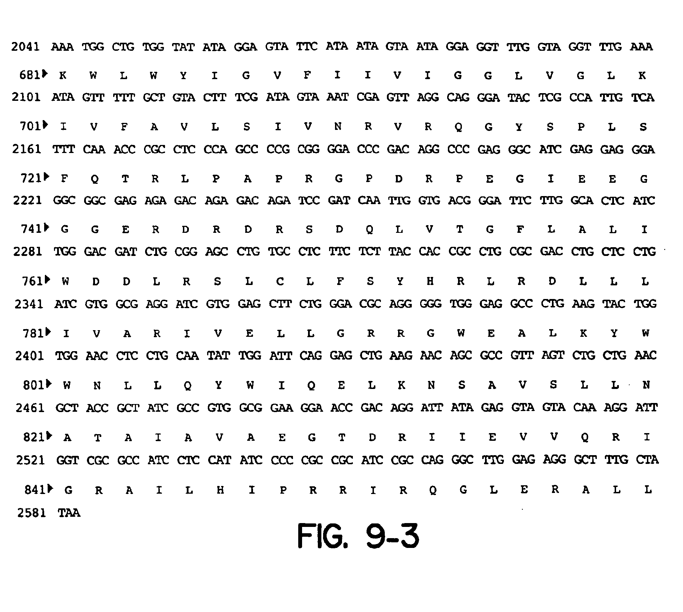

Similar DNA expression vectors were produced for HIV env protein (see, e.g., Figures 8-9), as well as for SIV gag and env proteins. The HIV env plasmids were constructed based on a HIV clade B env sequence and tested for expression. Expression was high in the absence of Rev. (See Figure 10). Specific vectors, and combinations thereof, are described in more detail below. We also have variations of the vectors that do not contain linker amino acids, or contain fewer amino acids for CATENIN, etc, which are not specifically exemplified herein. Smaller fragments of the secretory sequences, or the destabilization sequence, than those exemplified herein, which maintain the desired function, are in some cases known to exist, or can be identified by routine experimentation. These sequences are also useful herein.
p37gag = HIV plasmid described previously
MCP3p37gag = as above, plus also contains also the leader sequence of ip10

The following is an example for MCP3p37gag :

The vector pCMVkanMCP3gagp37M1-10 expresses the following MCP3-gag fusion protein (SEQ ID NO: 1): 
M N P S A A V I F C L I L L G L S G T Q
(IP10)
G I L D (linker)
M A Q P V G I N T S T T C C Y R F I N K K I P K
Q R L E S Y R R T T S S H C P R E A V I F K T K
L D K E I C A D P T Q K W V Q D F M K H L D K
K T Q T P K L (MCP-3)
A S A G A (linker)
G A R A S V L S G G E L D R W E K I R L R P G G
K K K Y K L K H I V W A S R E L E R F A V N P G
L L E T S E G C R Q I L G Q L Q P S L Q T G S E E
L R S L Y N T V A T L Y C V H Q R I E I K D T K
E A L D K I E E E Q N K S K K K A Q Q A A A D T
G H S N Q V S Q N Y P I V Q N I Q G Q M V H Q A
I S P R T L N A W V K V V E E K A F S P E V I P
M F S A L S E G A T P Q D L N T M L N T V G G H
Q A A M Q M L K E T I N E E A A E W D R V H P
V H A G P I A P G Q M R E P R G S D I A G T T S
T L Q E Q I G W M T N N P P I P V G E I Y K R W
I I L G L N K I V R M Y S P T S I L D I R Q G P K
E P F R D Y V D R F Y K T L R A E Q A S Q E V K
N W M T E T L L V Q N A N P D C K T I L K A L G
P A A T L E E M M T A C Q G V G G P G H K A R
V L E F • (p37gag HIV)
CYBp37gag = contains cyclin B destabilizing sequences
CATEp37gag = contains beta catenin destabilizing sequences
MOSp37gag = contains mos destabilizing sequences
SIVMCP3p39= as above for HIV
SIVCATEp39= as above for HIV

SIVgagDX is a Rev-independent SIV gag molecular clone. This vector is described in PCT/US00/34985 filed December 22, 2000 (published as WO 01/46408 on June 28, 2001). P39 denotes a DNA sequence encoding SIV Gag p39 (SIV p17 + p25). P57 denotes a DNA sequence encoding the complete SIV Gag p57.

"Gag" denotes DNA sequence encoding the Gag protein, which generates components of the virion core, "Pro" denotes "protease." The protease, reverse transcriptase, and integrase genes comprise the "pol" gene. In these constructs, "MCP3" denotes MCP-3 amino acids 33-109 linked to IP-10 secretory peptide refered supra (alternatively, it can be linked to its own natural secretory peptide or any other functional secretory signal such as the tPA signal mentioned supra), "CYB" denotes Cyclin B amino acids 10-95, "MOS" denotes C-Mos amino acid 1-35 and "CATE" denotes β-catenin amino acids 18-47.

Cyclin B nucleic acid sequences and encoded amino acids used in the constructs exemplified herein:

ATGTCCAGTGATTTGGAGAATATTGACACAGGAGT
TAATTCTAAAGTTAAGAGTCATGTGACTATTAGGC
GAACTGTTTTAGAAGAAATTGGAAATAGAGTTAC
AACCAGAGCAGCACAAGTAGCTAAGAAAGCTCAG
AACACCAAAGTTCCAGTTCAACCCACCAAAACAA
CAAATGTCAACAAACAACTGAAACCTACTGCTTCT
GTCAAACCAGTACAGATGGAAAAGTTGGCTCCAA
AGGGTCCTTCTCCCACACCTGTCGACAGAGAGATG
GGTGCGAGAGCGTCAGTATTAAGCGGGGGAGAAT
TAGATCGATGGGAAAAAATTCGGTTAAGGCCAGG
GGGAAAGAAGAAGTACAAGCTAAAGCACATCGTA
TG (SEQ ID NO: 2)

MetSerSerAspLeuGluAsnIleAspThrGlyValAsnSerLysVal
LysSerHisValThrIleArgArgThrValLeuGluGluIleGlyAsnAr
gValThrThrArgAlaAlaGlnValAlaLysLysAlaGlnAsnThrLy
sValProValGlnProThrLysThrThrAsnValAsnLysGlnLeuLy

sProThrAlaSerValLysProValGlnMetGluLysLeuAlaProLys
GlyProSerProThrProValAspArgGlu (SEQ ID NO: 3)

c-Mos nucleic acid sequences and encoded amino acids used in the constructs exemplified herein:
ATGCCCGATCCCCTGGTCGACAGAGAG (SEQ ID NO: 4)
MetProAspProLeuValAspArgGlu (SEQ ID NO: 5)

### EXAMPLE 2

### Construction of Vectors

In order to design "Gag-destabilized" constructs, a literature search for characterized sequences able to target proteins to the ubiquitin-proteasome degradation pathway gave the following, not necessarily representative, list:

| | |
|---|---|
| c-Myc | aa2-120 |
| Cyclin A | aa13-91 |
| Cyclin B | aa13-91 *we used 10-95 in vectors in examples herein |
| IkBa | aa20-45 |
| b-Catenin | aa19-44 *we used 18-47 in vectors in examples herein |
| c-Jun | aa1-67 |
| c-Mos | aa1-35 |

We cloned a subset of those degradation sequences from Jurkat cDNA, namely the signals from cyclin B, β-catenin, and c-Mos, using PCR. Both cyclin and catenin primers gave fragments of the expected length, that were cut and cloned into the SalI site of the vectors pCMV37(M1-10)kan, or pCMV55(M1-10)kan, and (Bam version) into the BamHI site of pFREDlacZ. (The p37 and p55 plasmids have the same p37 and p55 sequences disclosed in the patents containing INS- gag sequences (see, e.g., U.S. Patent No. 5,972,596 and U.S. Patent No. 5,965,726) but they have a different plasmid backbone expressing kanamycin. pFREDlacZ contains the IE CMV promoter expressing beta galactosidase of E coli.)

The corresponding plasmids are called:

| | |
|---|---|
| pCMV37(M1-10)kan with cyclin B sequence in SalI site | **pS194** |
| pCMV37(M1-10)kan with β-catenin sequence in SalI site | **pS195** |
| pCMV55(M1-10)kan with cyclin B sequence in SalI site | **pS199** |
| pCMV55(M1-10)kan with β-catenin sequence in SalI site | **pS200** |
| pFREDlacZ with cyclin B sequence in BamHI site | **pS201** |
| pFREDlacZ with β-catenin sequence in BamHI site | **pS202** |

In the case of Mos, the degradation signal consists of five N-terminal amino acids and a lysine approximately 30 amino acids away. A similarly located lysine is present in HIV gag, but not in lacZ. For that reason, oligos covering all five destabilizing amino acids were synthesized (both chains), annealed, and linked to the N-terminus of gag, but not lacZ. There were three versions of MOS sequence:

| | |
|---|---|
| MOSN5wtUP & MOSN5wtDN | has serine shown to cause degradation when phosphorylated |
| MOSN5aspUP & MOSN5aspDN | has Asp for Ser substitution, mimicking phosphorylation for constitutive action |
| MOSN5argUP & MOSN5argDN | has Arg for Ser substitution, allegedly making degradation signal inactive |

Out of six plasmids planned, we only examined the following:
**pS191** having pCMV37(M1-10)kan with the wild type ("WT") Mos sequence, but the insert is longer than intended, with an additional copy of the synthetic sequence in reverse;
**pS192** having pCMV37(M1-10)kan with "Asp" Mos sequence in the SalI site; and
**pS197** with pCMV55(M1-10)kan with "Asp" Mos sequence in the SalI site.

### EXAMPLE 3

### Preliminary Characterization of the Degradation Signals in the Vectors

The following experiments were conducted for preliminary characterization of the degradation signals in the nucleic acid constructs described above.

β-galactosidase activity was measured in transiently transfected HeLa and 293 cells after transfection with either pFREDlacZ or its cyclin B or β-catenin- modified versions (pS201 & 202). Apparent loss of the lacZ activity was interpreted as being indicative of ubiquitination signal-induced protein degradation.

With modified Gag the following experiments were done to confirm that degradation signals work in the gag context as well. First, p24-gag was measured by ELISA in cellular extracts and supernatants of cells transfected with the modified Gag constructs. Although we obtained evidence of destabilization, in several cases this experiment measuring the total level of p24 antigen was inconclusive. This was probably because, as shown previously, fragments of gag can still score positive in the antigen capture assay procedure. Therefore we looked into how intact the produced proteins were.

Protein extracts of HeLa or 293 cells transiently transfected with different gag plasmids were run on acrylamide tris-glycine gel, transferred to Immobilon P membrane and stained with anti-HIV antibodies to reveal Gag. These experiments did not show any signs of degradation in HeLa cells, however 293 cells transformed with the cyclin or β-catenin-modified versions of Gag clearly demonstrated the presence of prominent Gag-stained bands of molecular weight smaller than the full-length modified Gag. Such non-full length bands were not observed with the wild type Gag-transfected cells. These finding is consistent with the signal-induced Gag degradation.

To further examine whether the N-terminal modifications induce Gag degradation, we conducted pulse-chase experiments with transiently transfected 293 cells. One day after transfection the cells were incubated in methionine-free medium to exhaust cellular pools, labeled with ³⁵S-methionine in the same medium, and chased by adding 1000-fold excess of the cold methionine. Two experiments have been done. One with ~1 hour pulse and 12 hours chase, and another with 30 min pulse and 1.5 h chase. The experiments showed that the modified Gag degrades more rapidly than the wild type Gag. Both cyclin B and β-catenin-derived signals worked in destabilizing Gag to a similar extent. Additional experiments were performed with the env constructs-beta catenin fusions, and verified that the fusions were much more unstable after expression in human cells.

### EXAMPLE 4

### Proliferative Responses of Vectors And Combinations of Vectors

These vectors were tested for protein expression in vitro after transfections in mammalian cells and for immunogenicity in mice and primates (macaques).

### Methods:

DNA was purified using the Qiagen endotoxin free DNA purification kit. Endotoxin levels were routinely measured and were very low (kinetic-QCL test, Bio-Whittaker gave approximately 1 endotoxin unit/mg of DNA in these preparations).

Mice were injected intramuscularly with 100 µg of DNA in 100 µl of PBS. Three injections of DNA were given at days 0 14 and 28. At day 35 mice were sacrificed and their splenocytes assayed for proliferation in the presence of the specific gag antigen. In addition, cytotoxic responses were evaluated by performing standard cytotoxicity assays. The antibody response of the vaccinated mice is also under evaluation using sera obtained from these animals.

For monkey experiments, 5 mg of MCP3gag HIV DNA in 5 ml of phosphate buffered saline (PBS) were injected in several spots intramuscularly in Rhesus macaques, after the animals were sedated. Four injections were given at 0, 2, 4, and 8 weeks. The animals were followed by several assays to assess cellular and humoral immune response. Previous immunizations with gag p37M1-10, described in our previous patent gave only low levels of antibodies. The previous gag construct stimulated cellular immunity well, but not antibodies.

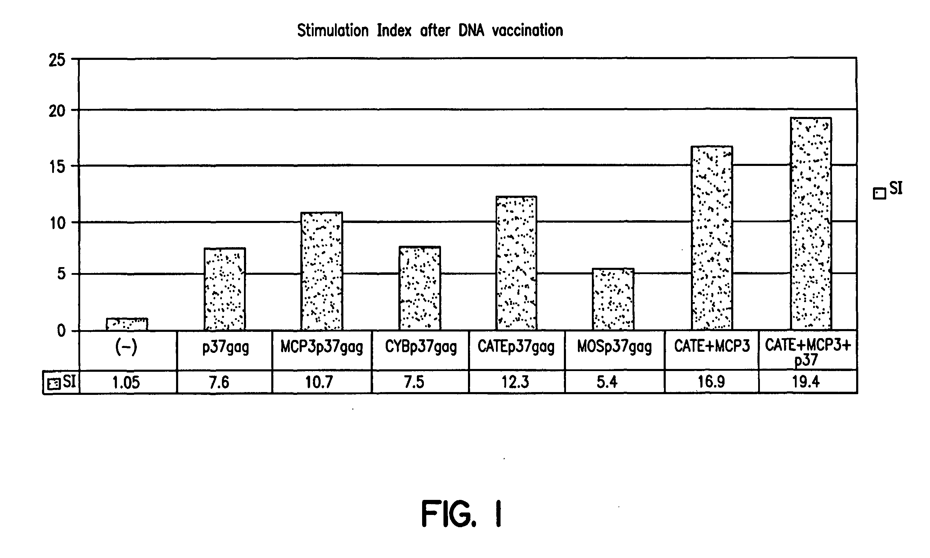

Figure 1 shows the proliferative responses (shown as stimulation index, SI) in mice injected with the indicated vectors or combinations of the following vectors containing DNA sequences encoding HIV polypeptides, or polypeptide controls:
p37gag
MCP3p37gag
CYBp37gag
CATEp37gag
MOSp37gag = *we used WT Mos in the example herein
CATE+MCP3 =*2 constructs, see above; these are the same plasmids used alone or in combinations
CATE+MCP3+p37 = *3 constructs, see above

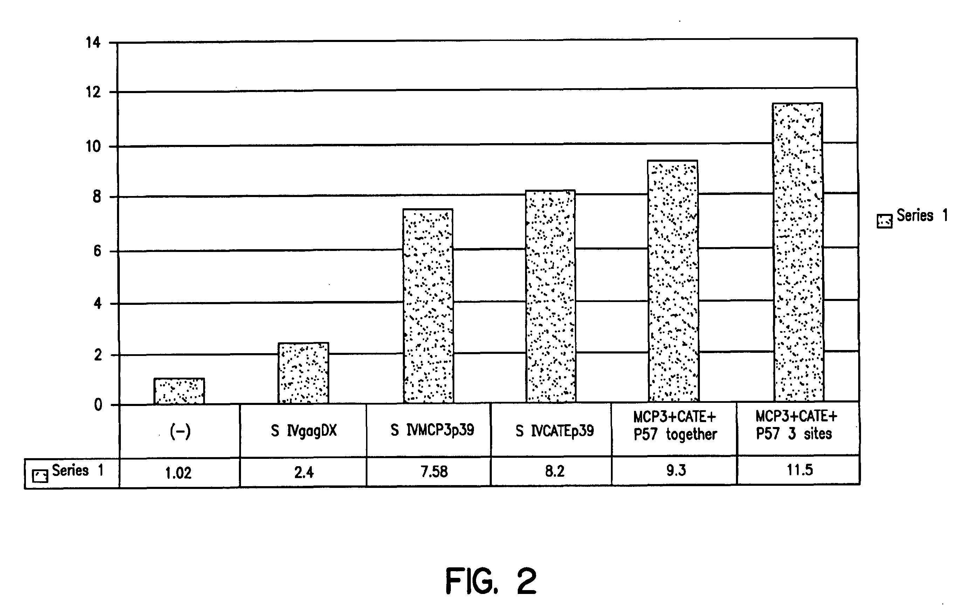

Figure 2 shows proliferative responses (shown as stimulation index, SI) in mice injected two times with the indicated SIV expression plasmids or combinations. Together = injection of 3 DNAs at the same sites; 3 sites = injections of the same DNAs at separate sites. When the "same sites" were used, all DNAs were mixed and injected at the same body sites in the muscle. When separate sites were used, the DNAs were kept separate and injected at anatomical sites that are separate. This happened every time we immunized the mice, i.e., the 3 DNAs were kept separate and injected at different sites from each other; and different sites of injection were used for each vaccination.
SIVgagDX
SIVMCP3p39
SIVCATEp39
MCP3+CATE+P57 (together)
MCP3+CATE+P57 (3 sites)

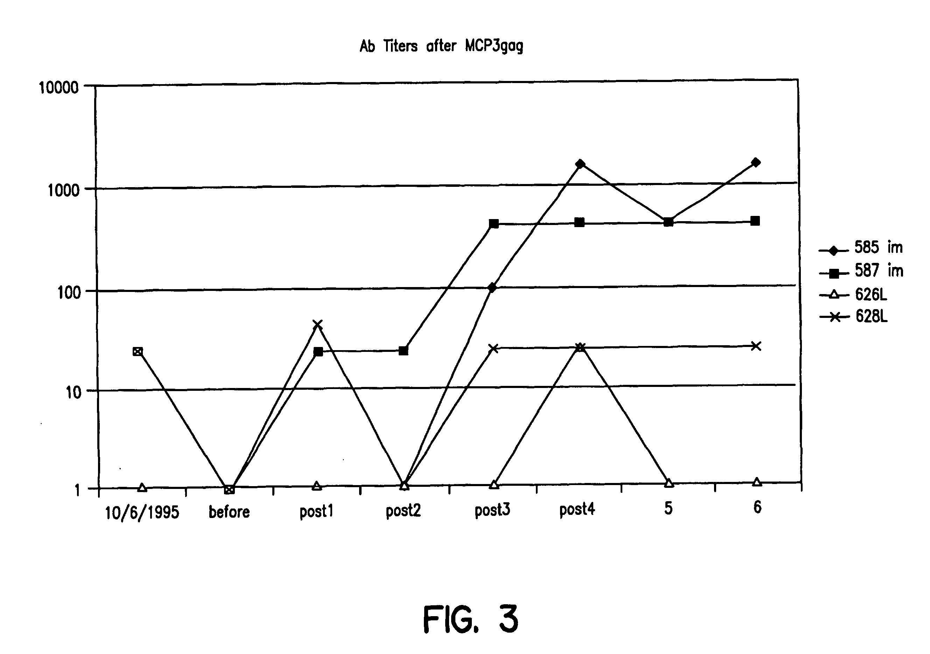

Figure 3 shows the antibody response in monkeys. Two animals (#585, 587) were injected 4x with 5 mg IM of MCP3p37gag expression vector. Two animals (#626, 628) were given the same DNA mucosally as liposome-DNA preparations. Titers plotted as reciprocal serum dilutions scoring positive in anti-HIV p24 Eliza tests.

### RESULTS

We found that MCP-3 fusions to gag dramatically increased the immune response to gag, compared to the unmodified gag vectors (type 1 as described above), see figures. This property may be in part the result of more efficient gag secretion from the cells, since we have recently shown that secreted gag having the leader sequence of tPA was more efficient in secretion and immunogenicity (Qiu et al, J. Virol. 2000).

In addition, this effect may be mediated by the function of MCP-3 molecule. The magnitude of the response suggests additional effects of MCP-3, in agreement with the reported effects of MCP-3 in inducing immunogenicity against a tumor antigen. Intramuscular injection of this MCP3p37gag in macaques led to the production of high titer anti-gag antibodies. This was not the case with previously tested gag expression vectors, indicating that it is possible to elicit an efficient antibody response in primates by only DNA vaccination. In addition, these results suggest that improved immunogenicity in mice was a satisfactory method to predict increased immunogenicity in primates. We therefore tested several vectors and combinations of vectors in mice, in an effort to identify the best combinations for subsequent experiments in primates.

We also studied the expression and immunogenicity of vectors that direct the expressed HIV antigens towards proteasome degradation and efficient presentation on the cell surface via the MHC-I class of molecules. MHC-Irestricted immunity is known to be important for anti-viral defenses. MHC-I display intracellularly produced short peptides on cell surface. A change in the composition of the peptides exposed by a cell, signals to the immune system that the cell is abnormal (e.g. virally infected) and should be destroyed. The MHC-I -- exposed peptides originate from proteasomal degradation of cellular proteins. We tested the hypothesis that supplying HIV antigens with strong additional ubiquitination signals targeting it for proteasomal degradation would increase its chances for being processed for surface presentation.

We tested several ubiquitination signals identified within known proteins for conferring rapid degradation after linking them to the N-terminus of HIV Gag. In parallel, the same ubiquitination signals were fused to beta-galactosidase to check for degradation efficiency by the drop in its enzymatic activity. This assay showed that all selected signals enhanced beta-galactosidase degradation.

The most effective sequence identified by these experiments corresponds to amino acids 18-47 of beta-catenin, a protein involved in Wnt signaling and cell-cell adhesion, whose abundance is controlled by degradation.

30 aa of Beta-catenin (18-47):

R K A A V S H W Q Q Q S Y L D S G I H S G A T T
T A P S L S (SEQ ID NO: 6)

Beta-catenin(18-47) added at the N terminus of HIV antigens with initiator AUG Met:

M R K A A V S H W Q Q Q S Y L D S G I H S G A
T T T A P S L S (SEQ ID NO: 7)

Injecting mice with DNA constructs expressing either HIV-I Gag, or Gag fused with beta-catenin destabilizing domain showed that the latter construct was more immunogenic. Compared with Gag alone, beta-catenin-Gag fusion evoked higher HIV-specific proliferative responses, elevated CTL response, and higher level of CD8+ IFNgamma+ -secreting cells.

Direct comparisons with other destabilizing sequences showed an overal higher potency of beta-catenin-Gag fusion. Therefore, one surprising conclusion is that, although several sequences increased proteasome processing and protein destabilization, the beta-catenin sequences were much better in inducing an increased immune response. Since the practical outcome of these studies is improved vaccination procedures, we propose the use of preferably the beta-catenin sequences identified here for use in targeting antigens for degradation.

Another important conclusion came from studies of combinations of vectors expressing different forms of antigens. It was found that combinations showed improved immunogenicity especially when injected in different sites on the same mouse, compared to a mix of DNA vectors injected in the same site.

We propose that different forms of the antigens trigger qualitatively different immune responses. Therefore, combinations of antigens applied at different sites and also at different times, may increase protective immune response. The results so far support the conclusion that using different forms of DNA sequentially or in combinations but applied at different sites may reproduce the good immunogenicity obtained with other prime-boost vaccine combinations. This will be a dramatic improvement over existing procedures for DNA vaccination in primates, which has been shown to be inefficient, especially for stimulating humoral immunity.

### EXAMPLE 5

### Immunogenicity of SIV Gag and SIV Env DNA Vectors in Macaques

On the basis of previous data suggesting that the modified forms of HIV and SIV antigens showed different immune responses after DNA vaccination, we studied the immunogenicity of three different DNA vaccine vectors for SIV gag and SIV env in 12 macaques. The DNAs used are shown in Table 1, below:

**Table 1**

| SIV DNA Vectors | | | |
|---|---|---|---|
| | **gag** | full name: | |
| 1 | p57gag | SIVgagDX | WT |
| 3 | MCP3gag | SIVMCP3p39 | extracellular |
| 5 | CATEgag | SIVCATEp39 | intra cellular |

| | **env** | | |
|---|---|---|---|
| 2 | gp160env | pCMVkan/R-R-SIVgp160CTE | WT |
| 4 | MCP3env | pCMVkan/MCP3/SIVgp160CTE | extracellular |
| 6 | CATEenv | pCMVkan/CATE/SIVgp160CTE | intra cellular |

The SIV gag vectors are the same as those used in the mice experiments described in the previous examples above. The SIVenv parent vector has been described in patent application serial no. 09/872,733, filed June 1, 2001 as an example of a vector with high levels of expression. The schematic diagram and sequence of this vector are set forth in Figures 6 and 7 herein, respectively. The MCP3 and CATE fusion vectors contain the same sequences of MCP3 and CATE described for the gag vectors.

Three groups of four naive macaques (groups 1, 2, 3) were immunized intramuscularly with purified DNA preparations in PBS as shown in Table 2:

**Table 2**

| DNA Immunization | | | | |
|---|---|---|---|---|
| week: | 0 | 4 | 12 | 24 |
| Group 1: | 1,2,3,4 | 1,2,3,4 | 1,2,3,4 | 1,2,3,4 |
| Group 2: | 1,2,5,6 | 1,2,5,6 | 1,2,5,6 | 1,2,5,6 |
| Group 3: | 1,2,3,4,5,6 | 1,2,3,4,5,6 | 1,2,3,4,5,6 | 1,2,3,4,5,6 |
| Group 4: | 5,6 | 5,6 | 3,4 | 3,4 |
| Group 5: | 1,2 | 1,2 | 1,2 | 1,2 |

The animals were injected with the indicated DNAs. The total amount of DNA injected each time per animal was kept constant at 3 mg for gag and 3 mg for env. Animals were injected at different sites with the different DNAs. Injections were intramuscularly with the DNA delivered in PBS at 1 mg/ml. The sites of injections were anatomically separate for the different DNAs.

In addition, four animals (group 4) were immunized first with DNAs 5 and 6 (i.e., SIV CATE gag and SIV CATE env), and subsequently at weeks 12 and 24 with DNAs 3 and 4 (i.e., SIV MCP3 gag and SIV MCP3 env). Two animals in group5 received the DNAs expressing unmodified, wild-type antigens for gag and env (1 and 2). The animals in groups 4 and 5 had been previously exposed to HIV DNA, but they were naive for SIV antigens, which was verified by immunological assays (Antibody measurements and lymphoproliferative responses to specific antigen stimulation). Despite this, animals in groups 4 and 5 showed early responses to SIV DNA injection, indicating an anamnestic response to SIV antigens. Therefore, the experiment for groups 4 and 5 needs to be repeated with naïve animals for final conclusions.

At sequential times during vaccination blood samples were obtained and analyzed for the presence of antibodies, lymphoproliferative responses and cytotoxic T cells.

The antibody titers obtained for gag are as shown in Table 3. The reciprocal of the highest dilution scoring positive in Elisa assays is shown. Empty cells indicate antibody reactivity below 1:50 dilution.

These results showed that administration of MCP3gag vector is associated with strong antibody response, because 8/8 (100%) of animals receiving MCP3gag (in Groups 1 and 3) developed high gag antibodies. In contrast, 3/6 (50%) of animals not receiving MCP3gag (in Groups 2 and 5) developed antibodies.

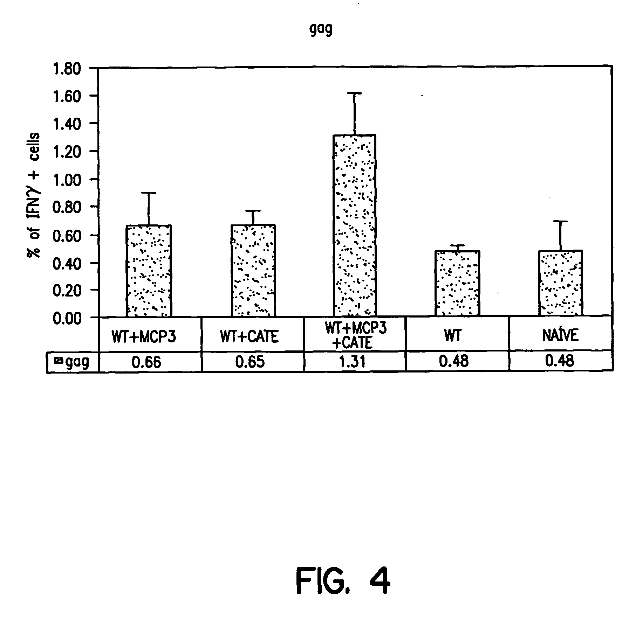

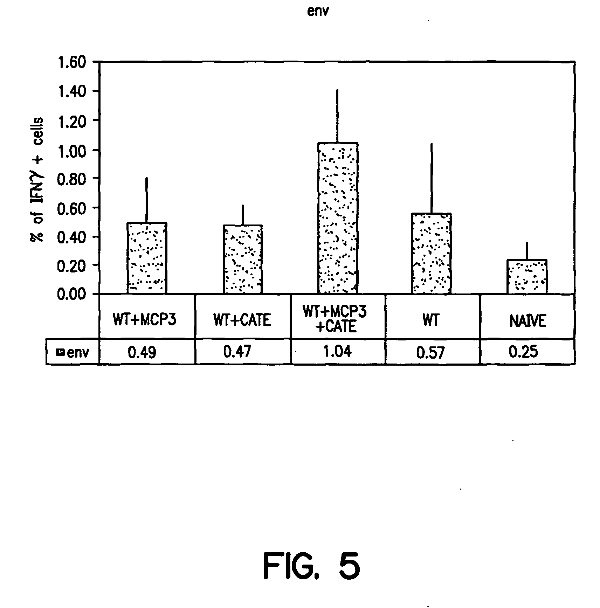

The specific cytotoxic T cell responses against gag and env were evaluated by measuring the number of CD8 cells that produce intracellular IFNgamma or TNFalpha in the presence of gag or env synthetic peptide pools (overlapping 15mers). The values obtained after three DNA vaccinations are shown in Figures 4 and 5. It is interesting that the combination of three vectors increased the number of specific IFNgamma-producing cells upon peptide stimulation. It was concluded that the animals receiving all three forms of antigens showed increased antibody response without diminishing cellular immune response. Actually the cellular immune response also showed increased cellular immune response and the results showed statistical significant differences.

These data indicate the development of a more balanced immune response than previously anticipated by DNA vaccination in macaques, by the combination of different antigen forms.

Group 4 responses (not shown above) were also elevated (1.11% and 0.88% for gag and env, respectively), but this needs to be repeated by vaccinating naive animals.

The mechanism of this increased immunogenicity by the combination of DNA vectors needs to be examined further. Expression and secretion of MCP-3-antigen chimeras may lead to increased protein levels that stimulate efficiently humoral immune responses. The combination of different antigen forms may also promote better activation and coordination of effector cells.

Table 3 shows SIV gag antibody response for all groups from the time of first immunization.

**Table 3**

| **Antibody Titers In Monkeys Vaccinated with SIV DNAs (Groups 1-5)** | | | | | | | | | | | | |
|---|---|---|---|---|---|---|---|---|---|---|---|---|
| | animal# | week | | | | | | | | | | |
| | | **0** | **3** | **4** | **6** | **8** | **12** | **13** | **14** | **24** | **25** | **26** |
| **Group1 WT+MCP3** | **918L** | | | | 50 | | 50 | 800 | 3200 | 50 | 800 | 12800 |
| | 919L | | | | | | | | | 50 | 50 | 3200 |
| | **921L** | | | | 50 | | | | | 50 | 50 | 800 |
| | **922L** | | | | | | | 800 | 3200 | 50 | 50 | 3200 |
| **Group2 WT+CATE** | **920L** | | | | | | | 200 | 800 | 50 | 50 | 50 |
| | **923L 924L 925L** | | | | | | | | 200 | 50 | 3200 | 3200 |
| **Group3 WT+MCP3 +CATE** | **926L** | | | | | | | 50 | 200 | 50 | 3200 | 3200 |
| | **927L** | | | | | | | | | 50 | 50 | 50 |
| | **928L** | | | | | | | 50 | 800 | 50 | 50 | 3200 |
| | **929L** | | | | | | | 50 | 200 | 50 | 3200 | 3200 |
| **Group4 CATE, then MCP3** | **585L** | | 800 | 800 | 3200 | 3200 | 800 | 3200 | 800 | 800 | 3200 | 3200 |
| | **587L** | | | 50 | 50 | | | 3200 | 3200 | 12800 | 3200 | 3200 |
| | **626L** | | 800 | 200 | 50 | | | | 50 | 50 | 3200 | 3200 |
| | **628L** | | | | | | | | | 50 | 50 | 3200 |
| **Group5 WT** | **715L** | | 50 | | | 800 | 200 | 200 | 200 | 50 | 50 | 3200 |
| | **716L** | | | | | | 800 | | | | | |

### EXAMPLE 6

### Use Of Nucleic Acids of the Invention In Immunoprophylaxis Or Immunotherapy

In postnatal gene therapy, new genetic information has been introduced into tissues by indirect means such as removing target cells from the body, infecting them with viral vectors carrying the new genetic information, and then reimplanting them into the body; or by direct means such as encapsulating formulations of DNA in liposomes; entrapping DNA in proteoliposomes containing viral envelope receptor proteins; calcium phosphate co-precipitating DNA; and coupling DNA to a polylysine-glycoprotein carrier complex. In addition, in vivo infectivity of cloned viral DNA sequences after direct intrahepatic injection with or without formation of calcium phosphate coprecipitates has also been described. mRNA sequences containing elements that enhance stability have also been shown to be efficiently translated in Xenopus laevis embryos, with the use of cationic lipid vesicles. See, e.g., J.A. Wolff, et al., Science 247:1465-1468 (1990) and references cited therein.

It has also been shown that injection of pure RNA or DNA directly into skeletal muscle results in significant expression of genes within the muscle cells. J.A. Wolff, et al., Science 247:1465-1468 (1990). Forcing RNA or DNA introduced into muscle cells by other means such as by particle-acceleration (N. -S. Yang, et al. Proc. Natl. Acad. Sci. USA 87:9568-9572 (1990); S.R. Williams et al., Proc. Natl. Acad. Sci. USA 88:2726-2730 (1991)) or by viral transduction or in vivo electorporation should also allow the DNA or RNA to be stably maintained and expressed. In the experiments reported in Wolff et al., RNA or DNA vectors were used to express reporter genes in mouse skeletal muscle cells, specifically cells of the quadriceps muscles. Protein expression was readily detected and no special delivery system was required for these effects. Polynucleotide expression was also obtained when the composition and volume of the injection fluid and the method of injection were modified from the described protocol. For example, reporter enzyme activity was reported to have been observed with 10 to 100 µl of hypotonic, isotonic, and hypertonic sucrose solutions, Opti-MEM, or sucrose solutions containing 2mM CaCl₂ and also to have been observed when the 10- to 100- µl injections were performed over 20 min. with a pump instead of within I min.

Enzymatic activity from the protein encoded by the reporter gene was also detected in abdominal muscle injected with the RNA or DNA vectors, indicating that other muscles can take up and express polynucleotides. Low amounts of reporter enzyme were also detected in other tissues (liver, spleen, skin, lung, brain and blood) injected with the RNA and DNA vectors. Intramuscularly injected plasmid DNA has also been demonstrated to be stably expressed in non-human primate muscle. S. Jiao et al., Hum. Gene Therapy 3:21-33 (1992).

It has been proposed that the direct transfer of genes into human muscle in situ may have several potential clinical applications. Muscle is potentially a suitable tissue for the heterologous expression of a transgene that would modify disease states in which muscle is not primarily involved, in addition to those in which it is. For example, muscle tissue could be used for the heterologous expression of proteins that can immunize, be secreted in the blood, or clear a circulating toxic metabolite. The use of RNA and a tissue that can be repetitively accessed might be useful for a reversible type of gene transfer, administered much like conventional pharmaceutical treatments. See J.A. Wolff, et al., Science 247:1465-1468 (1990) and S. Jiao et al., Hum. Gene Therapy 3:21-33 (1992).

It had been proposed by J.A. Wolff et al., supra, that the intracellular expression of genes encoding antigens might provide alternative approaches to vaccine development. This hypothesis has been supported by a recent report that plasmid DNA encoding influenza A nucleoprotein injected into the quadriceps of BALB/c mice resulted in the generation of influenza A nucleoprotein-specific cytotoxic T lymphocytes (CTLs) and protection from a subsequent challenge with a heterologous strain of influenza A virus, as measured by decreased viral lung titers, inhibition of mass loss, and increased survival. J. B. Ulmer et al., Science 259:1745-1749 (1993).

Therefore, it appears that the direct injection of RNA or DNA vectors encoding the viral antigen can be used for endogenous expression of the antigen to generate the viral antigen for presentation to the immune system without the need for self-replicating agents or adjuvants, resulting in the generation of antigen-specific CTLs and protection from a subsequent challenge with a homologous or heterologous strain of virus.

CTLs in both mice and humans are capable of recognizing epitopes derived from conserved internal viral proteins and are thought to be important in the immune response against viruses. By recognition of epitopes from conserved viral proteins, CTLs may provide cross-strain protection. CTLs specific for conserved viral antigens can respond to different strains of virus, in contrast to antibodies, which are generally strain-specific.

Thus, direct injection of RNA or DNA encoding the viral antigen has the advantage of being without some of the limitations of direct peptide delivery or viral vectors. See J.A. Ulmer et al., supra, and the discussions and references therein). Furthermore, the generation of high-titer antibodies to expressed proteins after injection of DNA indicates that this may be a facile and effective means of making antibody-based vaccines targeted towards conserved or non-conserved antigens, either separately or in combination with CTL vaccines targeted towards conserved antigens. These may also be used with traditional peptide vaccines, for the generation of combination vaccines. Furthermore, because protein expression is maintained after DNA injection, the persistence of B and T cell memory may be enhanced, thereby engendering long-lived humoral and cell-mediated immunity.

### Vectors for the immunoprophylaxis or immunotherapy against HIV-1

In one embodiment described herein, the nucleic acids of the invention will be inserted in expression vectors containing REV independent expression cassettes using a strong constitutive promoter such as CMV or RSV, or an inducible promoter such as HIV-1.

The vector will be introduced into animals or humans in a pharmaceutically acceptable carrier using one of several techniques such as injection of DNA directly into human tissues; electroporation (in vivo or ex vivo) or transfection of the DNA into primary human cells in culture (ex vivo), selection of cells for desired properties and reintroduction of such cells into the body, (said selection can be for the successful homologous recombination of the incoming DNA to an appropriate preselected genomic region); generation of infectious particles containing the *gag* gene, infection of cells *ex vivo* and reintroduction of such cells into the body; or direct infection by said particles in vivo.

Substantial levels of protein will be produced (and rapidly degraded in the situations where destabilization sequences are part of the encoded protein) leading to an efficient stimulation of the immune system.

In another embodiment described herein, the described constructs will be modified to express mutated Gag proteins that are unable to participate in virus particle formation. It is expected that such Gag proteins will stimulate the immune system to the same extent as the wild-type Gag protein, but be unable to contribute to increased HIV-1 production. This modification should result in safer vectors for immunotherapy and immunophrophylaxis.

### VII. REFERENCES

1. Borrow, P., H. Lewicki, B.H. Hahn, G.M. Shaw, and M.B. Oldstone. 1994. Virus-specific CD8+ cytotoxic T-lymphocyte activity associated with control of viremia in primary human immunodeficiency virus type I infection. J. Virol. 68:6103-6110.
2. Koup, R.A., J. T. Safrit, Y. Cao, C.A. Andrews, G. McLeod, W. Borkowsky, C. Farthing, and D. D. Ho. 1994. Temporal association of cellular immune responses with the initial control of viremia in primary human immunodeficiency virus type I syndrome. J. Virol. 68:4650-4655.
3. Pantaleo, G., J. F. Demarest, H. Soudeyns, C. Graziosi, F. Denis, J. W. Adelsberger, P. Borrow, M. S. Saag, G. M. Shaw, R. P. Sekaly, et al. 1994. Major expansion of CD8+ T cells with a predominant V beta usage during the primary immune response to HIV. Nature 370:463-467.
4. Musey, L., J. Hughes, T. Schacker, T. Shea, L. Corey, and M.J. McElrath. 1997. Cytotoxic-T-cell reponses, viral load, and disease progression in early human immunodeficiency virus type 1 infection. N. Engl. J. Med. 337:1267-1274.
5. Ogg, G.S., X. Jin, S. Bonhoeffer, P.R. Dunbar, M.A. Nowak, S. Monard, J.P. Segal, Y. Cao, S.L. Rowland-Jones, V. Cerundolo, A. Hurley, M. Markowitz, D.D. Ho, D.F. Nixon, and A.J. McMichael. 1998. Quantiation of HIV-1-specific cytotoxic T lymphocytes and plasma load of viral RNA. Science 279:2103-2106.
6. Aldhous, M.C., K.C. Watret, J.Y. Mok, A. G. Bird, and K.S. Froeber. 1994. Cytotoxic T lymphocyte activity and CD8 subpopulations in children at risk of HIV infection. Clin. Exp. Immunol. 97:61-67.
7. Langlade-Demoyen, P., N. Ngo-Giang-Huong, F. Ferchal, and E. Oksenhendler. 1994. Human immunodeficiency virus (HIV) Nef-specific cytotoxic T lymphocytes in noninfected heterosexual contact of HIV-infected patients. J. Clin. Investig. 93:1293-1297.
8. Rowland-Jones, S.L., D.F. Nixon, M.C. Aldous, F. Gotch, K. Ariyoshi, N. Hallam, J. S. Kroll, K. Froebel, and A. McMichael. 1993. HIV-specific cytotoxic T-cell activity in an HIV-exposed but uninfected infant. Lancet 341:860-861.
9. Rosenberg, E.S., J.M. Billingsley, A.M. Caliendo, S.L. Boswell, P.E. Sax, S. A. Kalamas, and B.D. Walker. 1997. Vigorous HIV-1-specific CD4+ T cell responses associated with control of viremia. Science 278:1447-1450.
10. Schwartz, D., U. Sharma, M. Busch, K. Weinhold, T. Matthews, J. Lieberman, D. Birx, H. Farzedagen, J. Margolick, T. Quinn, et al. 1994. Absence of recoverable infectious virus and unique immune responses in an asymptomatic HIV+ long-term survivor. AIDS Res. Hum. Retrovir. 10:1703-1711.
11. Durali, D., J. Morvan, F. Letourneur, D. Schmitt, N. Guegan, M. Dalod, S. Saragosti, D. Sicard, J.P. Levy, and E. Gomard. 1998. Cross-reactions between the cytotoxic T-lymphocyte responses of human immunodeficiency virus-infected African and European patients. J. Virol. 72:3547-3553.
12. McAdam, S., P. Kaleebu, P. Krausa, P. Goulder, N. French, B. Collin, T. Blanchard, J. Whitworth, A. McMichael, and F. Gotch. 1998. Cross-clade recognition of p55 by cytotoxic T lymphocytes in HIV-1 infection. AIDS 12:571-579.
13. Qiu, J.T., R. Song, M. Dettenhofer, C. Tian, T. August, B.K. Felber, G.N. Pavlakis, and X.F. Yu. 1999. Evaluation of novel human immunodeficiency virus type 1 Gag DNA vaccines for protein expression in mammalian cells and induction of immune responses. J. Virol. 73:9145-9152.
14. Schneider, R., M. Campbell, G. Nasioulas, B.K. Felber, and G.N. Pavlakis. 1997. Inactivation of the human immunodeficiency virus type 1 inhibitory elements allows Rev-independent expression of Gag and Gag/protease and particle formation. J. Virol. 71:4892-4903.
15. Schwartz, S., M. Campbell, G. Nasioulas, J. Harrison, B.K. Felber, and G.N. Pavlakis. 1992. Mutational inactivation of an inhibitory sequence in human immunodeficiency virus type 1 results in Rev-independent gag expression. J. Virol. 66:7176-7182.
16. Schwartz S., B.K. Felber, and G.N. Pavlakis. 1992. Distinct RNA sequences in the gag region of human immunodeficiency virus type 1 decrease RNA stability and inhibit expression in the absence of Rev protein. J. Virol. 66:150-159.
17. Donnelly, J.J., J.B. Ulmer, J.W. Shiver, and M.A. Liu. 1997. DNA vaccines. Annu. Rev. Immunol. 15:617-648.
18. Ulmer, J.B., R.R. Deck, C.M. Dewitt, J.I. Donnhly, and M.A. Liu. 1996. Generation of MHC class I-restricted cytotoxic T lymphocytes by expression of a viral protein in muscle cells: antigen presentation by non-muscle cells. Immunology. 89:59-67.
19. Qui, J-T., B. Liv, C. Tian, G.N. Pavlakis, and X.F. Yu. Enhancement of primary and secondary cellular immune responses against human immunodeficiency virus type 1 Gag by using DNA expression vectors that target Gag antigen to the secretory pathway. J. Virol. 74:5997-6005.
20. Lu, S., J.C. Santoro, D.H. Fuller, J.R. Haynes,a nd H.L. Robinson. 1995. Use of DNAs expressing HIV-1 Env and noninfectious HIV-1 particles to raise antibody responses in mice. Virology 209:147-154.
21. Chapman, B.S., R.M. Thayer, K.A. Vincent, and N.L. Haigwood. 1991. Effect of intron A from human cytomegalovirus (Towne) immediate-early gene on heterologous expression in mammalian cells. Nucleic Acids Res. 19:3979-3986.
22. Li, Z., A. Howard, C. Kelley, G. Delogu, F. Collins and S. Morris. 1999. Immunogenicity of DNA vaccines expressing tuberculosis proteins fused to tissue plasminogen activator signal sequences. Infect. Immun. 67:4780-4786.
23. Lewis, P.J., S. van Drunen Little-van den Hurk, and L.A. Babiuk. 1999. Altering the cellular location of an antigen expressed by a DNA-based vaccine modulates the immune response. J. Virol. 73:10214-10223.
24. Ulmer, J.B., J.J. Donnelly, S.E. Parker, G.H. Rhodes, P.L. Felgner, V.J. Dwarki, S.H. Gromkowski, R.R. Deck, C.M. DeWitt, A. Friedman, et al. 1993. Heterologous protection against influenza by injection of DNA encoding a viral protein. Science 259:1745-1749.
25. Schneider, J., S.C. Gilbert, T.J. Blanchard, T. Hanke, K.J. Robson, C.M. Hannan, M. Becker, R. Sinden, G.L. Smith, and A.V. Hill. 1998. Enhanced immunogenicity for CD8+ T cell induction and complete protective efficacy of malaria DNA vaccination by boosting with modified vaccinia virus Ankara. Nat. Med. 4:397-402.
26. Sedegah, M., T.R. Jones, M. Kaur, R. Hedstrom, P. Hobart, J.A. Tine and S.L. Hoffman. 1998. Boosting with recombinant vaccinia increases immunogenicity and protective efficacy of malaria DNA vaccine. Proc. Natl. Acad. Sci. USA 95:7648-7653.
27. Hanke, R., R.V. Samuel, T.J. Blanchard, V.C. Neumann, T.M. Allen, J.E. Boyson, S.A. Sharpe, N. Cook, G.L. Smith, D.I. Watkins, M.P. Cranage, and A.J. McMichael. 1999. Effective induction of simian immunodeficiency virus-specific cytotoxic T lymphocytes in macaques by using a multiepitope gene and DNA prime-modified vaccinia virus Ankara boost vaccination regimen. J. Virol. 73:7524-7532.
28. Robinson, H.L., D.C. Montefiori, R.P. Johnson, K.H. Manson, M.L. Kalish, J.D. Lifson, T.A. Rizvi, S. Lu, S.L. Hu, G.P. Mazzara, D.L. Panicali, J.G. Herndon, R. Glickman, M.A. Candido, S.L. Lydy, M.S. Wyand, and H.M. McClure. 1999. Neutralizing antibody-independent containment of immunodeficiency virus challenges by DNA priming and recombinant pox virus booster immunizations. Nat. Med. 5:526-534.
29. Bianchi, A., Massaia M. Idiotypic vaccination in B-cell malignances. Mol. Med. Today. 1997. 3:435-441
30. Chen TT, Tao MH, Levy R. Idiotype-cytokine fusion proteins as cancer vaccines. Relative efficacy of IL-2, IL-4, and granulocyte-macrophage colongy-stimulating factor. J. Immunol. 1994. 153:4775-4787.
31. Kwak LW, Young HA, Pennington RW, Week, SD. Vaccination with syngeneic, lymphoma-derived immunoglobulin idiotype combined with granulocyte/macrophage colongy-stimulating factor primes mice for a protective T-cell response. Proc. Natl. Acad. Sci. USA. 1996. 93:10972-10977.
32. Biragyn A., Tani K, Grimm, MC, Weeks, SD, Kwak LW. Genetic fusion of chemokines to a self tumor antigen induces protective, T-cell dependent antitumor immunity. Nat. Biotechnol. 1999. 17:253-258.
33. Kwak, LW, Campbell, MJ, Czerwinski, DK, Hart, S, Miller RA, Levy R. Induction of immune responses in patients with B-cell lymphoma against the surface-immunoglobulin idiotype expressed by their tumors. N. Engl. J. Med. 1992. 327:1209-1215.
34. Biragyn A, Kwak LW. B-cell malignancies as a model for cancer vaccines: from prototype protein to next generation genetic chemokine fusions. Immunol. Rev. 1999. Aug; 170:115-126.
35. Tobery, T. and R.F. Siliciano. Cutting Edge: induction of enhanced CTL-dependent protective immunity in vivo by N-end rule targeting of a model tumor antigen. J. Immunol. 1999. 162:639-642.
36. Tobery, T.W. and R.F. Siliciano. Targeting of HIV-1 antigens for rapid intracellular degradation enhances cytotoxic T lymphocyte (CTL) recognition and the induction of de novo CTL responses in vivo after immunization. 1997. J. Exp. Med. 185:909-920.
37. Goth, S., V. Nguyen, and N. Shastri. 1996. Geneartion of naturally procesed peptide/MHC class I complexes is independent of the stability of endogenously synthesized precursors. J. Immunol. 157:1894.
38. Minev, B.R., B.J. McFarland, P.J. Spiess, S.A. Rosenberg, and N.P. Restifo. 1994. Insertion signal sequence fused to minimal peptides elicits specific CD8+ T-cell responses and prolongs survival of thymoma-bearing mice. Cancer Res. 54:4155.
39. Rogers, W.O., K. Gowda, and S.L. Hoffman. 1999. Construction and immunogenicity of DNA vaccine encoding four Plasmodium vivax candidate vaccine antigens. Vaccine 17:3136-3144.

U.S. Patent No. 5,972,596 issued October 26, 1999 (Pavlakis and Felber)
U.S. Patent No. 5,965,726 issued October 12, 1999 (Pavlakis and Felber)
U.S. Patent No. 5,891,432 issued April 6, 1999 (Hoo).
U.S. Patent No. 6,100,387 issued August 8, 2000 (Hermanna nd Swanberg)
WO 98/17816 Lentiviral Vectors (Kingsman & Kingsman) (Oxford Biomedica Ltd)
WO 98/34640 (Shiver, J.W., Davies, M-E M., Freed, D.C., Liu, M.A. and Perry,H.C. - Merck & Co., Inc.)
WO 98/46083 Use of Lentiviral Vectors for Antigen Presentation in Dendritic Cells (Wong-Staal, Li; Kan-Mitchell) (Univ. of Cal.)
WO 99/04026 Lentiviral Vectors (Chen, Gasmi, Yee and Jolly) (Chiron)
WO 99/15641 Non-Primate Lentiviral Vectors and Packaging Systems (Poeschla, Looney and Wong-Staal) (Univ. of Cal.)
WO 99/30742 Therapeutic Use of Lentiviral Vectors (Naldini and Song)
WO 99/51754 Infectious Pseudotyped Lentiviral Vectors Lacking Matrix Protein and Uses Thereof (Goettlinger, Reil and Bukovsky) (Dana Farber Cancer Inst Inc)
PCT/US99/11082 Post-Transcriptional Regulatory Elements and Uses Thereof (Pavlakis and Nappi), filed May 22, 1999
Akkina, R.K., Walton, R.W., Chen, M.L., Li, Q-X, Planelles, V and Chen, I.S.Y., "High-efficiency gene transfer into CD34+ cells with a human immunodeficiency virus type 1-based retroviral vector pseudotyped with vesicular stomatitis virus envelope glycoprotein G," J. Virol. 70:2581-2585 (1996)
Amado, R.G. & Chen, I.S.Y., "Letinviral vectors-the promise of gene therapy within reach?," Science 285:674-676 (July 1999)
Donahue, R.E., An, D.S., Wersto, R.P., Agricola, B.A., Metzger, M.E. and Chen, I.S.Y., "Transplantation of immunoselected CD34+ cells transduced with a EGFP-expressing lentiviral vector in non-human primates," Blood 92(suppl. 1):383b, Abstract #4648.5 (1998)
Fox, J.L., "Researchers wary of fear-based ban on lentivirus gene therapy, " Nature Biotechnology 16:407-408 (1998)
Goldman, M.J., Lee, P.S., Yang, J.S. & Wilson, J.M., "Lentiviral vectors for gene therapy of cystic fibrosis," Hum Gene Ther. 8, 2261-2268 (1997)
Hartikka J, Sawdey M, CorNefert-Jensen F, Margalith M, Barnhart K, Nolasco M, Vahlsing HL, Meek J, Marquet M, Hobart P, Norman J, and Manthorpe M., "An improved plasmid DNA expression vector for direct injection into skeletal muscle," Hum Gene Ther. 7:1205-17 (1996)
Kafri, T., Blomer, U., Peterson, D.A., Gage, F.H. & Verma, I.M., "Sustained expression of genes delivered directly into liver and muscle by lentiviral vectors," Nat Genet. 17, 314-317 (1997)
Kafri, T., van Praag, H., Ouyang, L., Gage, F.G. and Verma, I.M., "A packaging cell line for lentivirus vectors," J Virol. 73:576-584 (1999)
Kim, V.N., Mitrophanous, K., Kingsman, S.M., and Kingsman, A.J., "Minimal Requirement for a Lentivirus Vector Based on Human Immunodeficiency Virus Type I", J. Virol. 72:811-816 (1998)
Klimatcheva, E., Rosenblatt, JD. and Planelles, V., "Lentiviral vectors and gene therapy," Frontiers in Bioscience 4:d481-496 (June 1999)
Miyoshi, H., Takahashi, M., Gage, F.H. & Verma, I.M., "Stable and efficient gene transfer into the retina using an HIV-based lentiviral vector," Proc Natl Acad Sci USA. 94: 10319-10323 (1997)
Miyoshi, H., Blomer,U., Takahashi, M., Gage, F.H., and Verma, I.M., "Development of self-inactivating lentivirus vector," ," J Virol. 72:8150-8157 (1998)
Miyoshi, H., Smith, K.A., Mosier, D.E., Verma, I.M. and Torbett, B.E., "Transduction of human CD34+ cells that mediate long-term engraftment of NOD/SCID mice by HIV vectors," Science 283:682-686 (1999)
Naldini, L., Blomer, U., Gallay, P., Ory, D., Mulligan, R., Gage, F.H., Verma, I.M. & Trono, D., "In vivo gene delivery and stable transduction of nondividing cells by a lentiviral vector," Science. 272, 263-267 (1996)
Naviaux, R.K, Costanzi, E., Haas, M. and Verma, I., "The pCL vector system: rapid production of helper-free, high-titer, recombinant retroviruses," J. Virol. 70:5701-5705 (1996)
Pavlakis, G.N., Schneider, R.; Song, S., Nasioulas, G., Zolotukhin, A., Felber, B.K., Trauger, R., Cox, J., and Manthorpe, M., "Use of simple Rev-independent HIV-1 gag expression vectors in gene therapy and gene vaccine applications," Natl Conf Hum Retroviruses Relat Infect (2nd), Jan 29-Feb 2 (1995); 91.
Poeschla, E.M., Wong-Staal, F. & Looney, D.J., "Efficient transduction of nondividing human cells by feline immunodeficiency virus lentiviral vectors," Nature Med 4:354-357 (1998)
Qiu, J. T., R. Song, M. Dettenhofer, C. Tian, T. August, B. K. Felber, G. N. Pavlakis and X. F. Yu, "Evaluation of novel human immunodeficiency virus type 1 Gag DNA vaccines for protein expression in mammalian cells and induction of immune responses," J Virol. 73: 9145-52 (Nov. 1999)
Reynolds, P.N. and Curiel, D.T., "Viral vectors show promise in Colorado," Nature Biotechnology 16:422-423 (1998)
Schneider, R., Campbell, M., Nasioulas, G., Felber, B.K., and Pavlakis, G.N., Inactivation of the human immunodeficiency virus type 1 inhibitory elements allows Rev-independent expression of Gag and Gag/protease and particle formation, "J. Virol. 71:4892-4903 (1997)
Schwartz, S., M. Campbell, G. Nasioulas, J. Harrison, B. K. Felber and G. N. Pavlakis, "Mutational inactivation of an inhibitory sequence in human immunodeficiency virus type-1 results in Rev-independent gag expression," J. Virol. 66:7176-7182 (1992)
Shiver, J.W., Yasutomi, Y., Free, D.C., Davies, M.-E., Perry, H.C., Pavlakis, G.N., Letvin, N.L., and Liu, M.A., "DNA Vaccine-Mediated Cellular Immunity Against HIV-1 gag and env", presented at the Conference on Advances in AIDS Vaccine Development: 8th Annual Meeting of the National Cooperative Vaccine Development Groups for AIDS (NCVDGs) from February 11-15, 1996.
Soneoka, Y., Cannon, P.M., Ransdale, E.E., Griffiths, J.C., Romano, G., Kingsman, S.M. and Kingsman, A.J., "A transient three-plasmid expression system for the production of high titer retroviral vectors," Nuc. Acids Res. 23:628-633 (1995).
Srinivasakumar, N., Chazal, N., Helga-Maria, C., Prasad, S., Hammarskjöld, M.-L., and Rekosh, D., "The Effect of Viral Regulatory Protein Expression on Gene Delivery by Human Immunodeficiency Virus Type 1 Vectors Produced in Stable Packaging Cell Lines," J. Virol., 71:5841-5848 (1997)
Sutton, R.E., Wu, H.T., Rigg, R., Bohnlein, E. & Brown, P.O., "Human immunodeficiency virus type 1 vectors efficiently transduce human hematopoietic stem cells," J Virol. 72, 5781-5788 (1998)
Tabemero, C., A. S. Zolotukhin, J. Bear, R. Schneider, G. Karsenty and B. K. Felber, "Identification of an RNA sequence within an intracisternal-A particle element able to replace Rev-mediated posttranscriptional regulation of human immunodeficiency virus type 1," J Virol. 71:95-101 (1997).. (see also my email message)
Takahashi, M.; Miyoshi, H.; Verma, I.M.; Gage, F.H., "Rescue from photoreceptor degeneration in the rd mouse by human immunodeficiency virus vector-mediated gene transfer," J. Virol. 73: 7812-7816 (Sept. 1999)
Uchida, N., Sutton, R.E., Friera, A.M., He, D., Reitsma, M.J., Chang, W.C., Veres, G., Scollay, R. & Weissman, I.L., "HIV, but not murine leukemia virus, vectors mediate high efficiency gene transfer into freshly isolated G0/G1 human hematopoietic stem cells," Proc. Natl Acad. Sci. USA. 95, 11939-11944 (1998)
Valentin, A., W. Lu, M. Rosati, R. Schneider, J. Albert, A. Karlsson and G. N. Pavlakis. "Dual effect of interleukin 4 on HIV-1 expression: Implications for viral phenotypic switch and disease progression," Proc. Natl Acad. Sci. U S A. 95: 8886-91 (1998)
White, S.M., Renda, M, Nam, N-Y, Klimatcheva, E., Hu, Y, Fisk, J, Halterman, M, Rimel, B.J., Federoff, H, Pandya, S., Rosenblatt, J.D. and Planelles, V, "Lentivirus vectors using human and simian immunodeficiency virus elements," J. Virol. 73:2832-2840 (Apr. 1999)
Wolff, J.A. and Trubetskoy, V.S., "The Cambrian period of nonviral gene delivery," Nature Biotechnology 16:421-422 (1998)
Zolotukhin, J., Valentin, A., Pavlakis, G. N. and Felber, B. K. "Continuous propagation of RRE(-)and Rev(-)RRE(-) human immunodeficiency virus type 1 molecular clones containing a cis-acting element of Simian retrovirus type 1 in human peripheral blood lymphocytes," J Virol. 68:7944-7952 (1994)
Zufferey, R., Nagy, D., Mandel, R.J., Naldini, L. and Trono, D., "Multiply Attenuated Lentiviral Vector Achieves Efficient Gene-Delivery In Vivo", Nature Biotechnology 15:871-875 (1997)
Zufferey, R., Dull, T., Mandel, R.J., Bukovsky, A., Quiroz, D., Naldini, L. & Trono, D., "Self-inactivating lentivirus vector for safe and efficient in vivo gene delivery," J Virol. 72:9873-9880 (1998)

Those skilled in the art will recognize that any gene encoding a mRNA containing an inhibitory/instability sequence or sequences can be modified in accordance with the exemplified methods or their functional equivalents.

Modifications of the above described modes for carrying out the invention that are obvious to those of skill in the fields of genetic engineering, virology, immunology, medicine, and related fields are intended to be within the scope of the following claims.

## Claims

1. A composition comprising one or more vectors expressing:
(i) a nucleic acid construct containing nucleotide sequences encoding a fusion protein comprising a destabilizing amino acid sequence that targets protein to the ubiquitin-proteasome degradation pathway covalently attached to an antigen, in which the immunogenicity of the antigen is increased by the presence of the destabilizing amino acid sequence, and
(ii) a nucleic acid construct encoding a secreted fusion protein comprising a MCP-3 chemokine amino acid sequence covalently attached to the antigen, in which the immunogenicity of the antigen is increased by the presence of the MCP-3 chemokine amino acid sequence.

2. A composition of claim 1 wherein the destabilizing amino acid sequence of (i) is present in an amino acid sequence selected from the group consisting of c-Myc aa2-120; Cyclin A aa13-91; Cyclin B 10-95; Cyclin B aa13-91; IkBa aa20-45; β-Catenin aa19-44; c-Jun aa1-67; and c-Mos aa1-35.

3. A composition of claim 1 wherein the destabilizing sequence is selected from the group consisting of c-Mos aa1-35; cyclin B aa 10-95; β-catenin 19-44 and β-catenin 18-47.

4. A composition of claim 1 wherein the destabilizing sequence is selected from β-Catenin 19-44 and β-catenin 18-47.

5. A composition of any one of the preceding claims wherein the MCP-3 chemokine amino acid sequence is MCP-3 amino acids 33-109 or 1-109.

6. A composition of any one of the preceding claims wherein the antigen is a disease associated antigen.

7. A composition of claim 6 wherein the disease associated antigen is selected from the group consisting of tumor-associated antigen, autoimmune disease-associated antigen, infectious disease-associated antigen, viral antigen, parasitic antigen and bacterial antigen.

8. A composition of claim 7 wherein the viral antigen is an HIV antigen.

9. A composition of claim 8 wherein said HIV antigen is selected from the group consisting of Gag, Env, Pol, Nef, Vpr, Vpu, Vif, Tat and Rev.

10. A composition of claim 6 wherein the disease associated antigen comprises antigenic fragments of HIV Gag-Pol-Tat-Rev-Nef or Tat-Rev-Env-Nef linked together, not necessarily in that order.

11. A composition of claim 1 wherein the nucleic acid construct encoding the secreted fusion protein is selected from the group consisting of (a) a construct comprising a sequence encoding HIV p37 gag, a MCP-3 chemokine sequence and a sequence of IP10 and (b) a construct comprising a sequence encoding SIV p39 gag, a MCP-3 chemokine sequence and a leader sequence of IP10.

12. A composition of claim 1 comprising:
(i) one or more vectors which encode wild-type Gag, Gag linked to MCP-3 amino acids 33-109 linked to IP-10 secretory peptide MNPSAAVIFCLILLGLSGTQ, and Gag linked to β-catenin amino acids 18-47; or
(ii) one or more vectors which encode wild-type Env, Env linked to MCP-3 amino acids 33-109 linked to IP-10 secretory peptide MNPSAAVIFCLILLGLSGTQ, and Env linked to β-catenin amino acids 18-47.

13. A composition of any one of the preceding claims, wherein the one or more vectors are contained in virus particles.

14. A pharmaceutical composition comprising one or more vectors as defined in any one of claims 1 to 12 and a pharmaceutically acceptable carrier.

15. A pharmaceutical composition of claim 14 for use in inducing antibodies in a mammal wherein said nucleic acid constructs are present in said composition in an amount which is effective to induce said antibodies in said mammal.

16. A pharmaceutical composition of claim 14 for use in inducing cytotoxic and/or helper-inducer T lymphocytes in a mammal wherein said nucleic acid constructs are present in said composition in an amount which is effective to induce cytotoxic and/or helper-inducer T lymphocytes in said mammal.

17. A vaccine composition for use in inducing immunity in a mammal against HIV infection comprising a therapeutically effective amount of one or more vectors as defined in any one of claims 8 to 12 and a pharmaceutically acceptable carrier.

18. A pharmaceutical composition comprising viral particles as defined in claim 13.

19. A pharmaceutical composition of claim 14 or 18 for use in stimulating an immune response against an antigen.

20. A composition of any one of claims 1 to 13 for use in vaccinating a mammal by inducing antibodies in the mammal, wherein said vectors are present in an amount which is effective to induce said antibodies in said mammal.

21. A composition of any one of claims 1 to 13 for use in vaccinating a mammal by inducing cytotoxic and/or helper-inducer T lymphocytes in the mammal, wherein said vectors are present in an amount which is effective to induce cytotoxic and/or helper-inducer T lymphocytes in said mammal.

22. A kit comprising one or more vectors as defined in any one of claims 1 to 12.

23. A first vector for use in a method for vaccinating a mammal by inducing antibodies, or cytotoxic and/or helper-inducer lymphocytes, wherein said method further comprises administration of a second vector,
wherein one of the vectors expresses a nucleic acid construct containing nucleotide sequences encoding a fusion protein comprising a destabilizing amino acid sequence that targets protein to the ubiquitin-proteasome degradation pathway covalently attached to an antigen in which the immunogenicity of the antigen is increased by the presence of the destabilizing amino acid sequence; and
the other vector expresses a nucleic acid construct encoding a secreted fusion protein comprising a MCP-3 chemokine amino acid sequence covalently attached to the antigen, in which the immunogenicity of the antigen is increased by the presence of the MCP-3 chemokine amino acid sequence.

24. The first vector for use in the method of claim 23, wherein in said one vector that expresses a nucleic acid construct containing nucleotide sequences encoding a fusion protein comprising a destabilizing amino acid sequence, said destabilizing amino acid sequence is selected from the group consisting of c-Myc aa2-120; Cyclin A aa13-91; Cyclin B 10-95; Cyclin B aa13-91; IkBa aa20-45; β-Catenin aa19-44; c-Jun aa1-67; and c-Mos aa1-35.

25. The first vector for use in the method of claim 23 or 24 wherein said vector is administered at the same site as the second vector.

26. The first vector for use in the method of claim 23 or 24 wherein said vector is administered at a different site from the second vector.

27. The first vector for use in the method of claim 23 or 24 wherein said vector is administered at the same time as the second vector.

28. The first vector for use in the method of claim 23 or 24 wherein said vector is administered at a different time from the second vector.

29. The first vector for use in the method of claim 23 or 24 wherein the antigen is an HIV antigen.

30. The first vector for use in the method of claim 23 or 24 wherein the antigen is HIV gag.

## Patentansprüche

1. Zusammensetzung, die einen oder mehrere Vektoren umfasst, der/die exprimiert/exprimieren:
(i) ein Nucleinsäurekonstrukt, das Nucleotidsequenzen enthält, die ein Fusionsprotein codieren, das eine destabilisierende Aminosäuresequenz, die Protein zu dem Ubiquitin-Proteasom-Abbauweg targetiert, kovalent an ein Antigen gebunden umfasst, wobei die Immunogenität des Antigens durch das Vorliegen der destabilisierenden Aminosäuresequenz erhöht ist, und
(ii) ein Nucleinsäurekonstrukt, das ein sekretiertes Fusionsprotein codiert, welches eine MCP-3-Chemokin-Aminosäuresequenz kovalent an das Antigen gebunden umfasst, wobei die Immunogenität des Antigens durch das Vorliegen der MCP-3-Chemokin-Aminosäuresequenz erhöht ist.

2. Zusammensetzung gemäß Anspruch 1, wobei die destabilisierende Aminosäuresequenz von (i) in einer Aminosäuresequenz vorliegt, die aus der Gruppe, bestehend aus c-Myc-aa2-120, Cyclin-A-aa13-91; Cyclin-B-10-95; Cyclin-B-aa13-91; IkBa-aa20-45; β-Catenin-aa19-44; c-Jun-aa1-67 und c-Mos-aa1-35, ausgewählt ist.

3. Zusammensetzung gemäß Anspruch 1, wobei die destabilisierende Sequenz aus der Gruppe, bestehend aus c-Mos-aa1-35; Cyclin-B-aa10-95; β-catenin-19-44 und β-catenin-18-47, ausgewählt ist.

4. Zusammensetzung gemäß Anspruch 1, wobei die destabilisierende Sequenz aus β-Catenin-19-44 und β-Catenin-18-47 ausgewählt ist.

5. Zusammensetzung gemäß einem der vorangehenden Ansprüche, wobei die MCP-3-Chemokin-Aminosäuresequenz MCP-3-Aminosäuren 33-109 oder 1-109 ist.

6. Zusammensetzung gemäß einem der vorangehenden Ansprüche, wobei das Antigen ein mit Krankheit assoziiertes Antigen ist.

7. Zusammensetzung gemäß Anspruch 6, wobei das mit Krankheit assoziierte Antigen aus der Gruppe, bestehend aus mit Tumor assoziiertem Antigen, mit Autoimmunerkrankung assoziiertem Antigen, mit Infektionskrankheit assoziiertem Antigen, viralem Antigen, parasitärem Antigen und bakteriellem Antigen, ausgewählt ist.

8. Zusammensetzung gemäß Anspruch 7, wobei das virale Antigen ein HIV-Antigen ist.

9. Zusammensetzung gemäß Anspruch 8, wobei das HIV-Antigen aus der Gruppe, bestehend aus Gag, Env, Pol, Nef, Vpr, Vpu, Vif, Tat und Rev, ausgewählt ist.

10. Zusammensetzung gemäß Anspruch 6, wobei das mit Krankheit assoziierte Antigen antigene Fragmente von HIV-Gag-Pol-Tat-Rev-Nef oder -Tat-Rev-Env-Nef miteinander verknüpft, nicht notwendigerweise in dieser Reihenfolge, umfasst.

11. Zusammensetzung gemäß Anspruch 1, wobei das Nucleinsäurekonstrukt, das das sekretierte Fusionsprotein codiert, ausgewählt ist aus der Gruppe, bestehend aus (a) einem Konstrukt, das eine Sequenz, die HIV-p37-gag, eine MCP-3-Chemokin-Sequenz und eine Sequenz von IP10 codiert, und (b) einem Konstrukt, umfassend eine Sequenz, codierend SIV-p39-gag, eine MCP-3-Chemokin-Sequenz und eine Leader-Sequenz von IP10.

12. Zusammensetzung gemäß Anspruch 1, umfassend:
(i) einen oder mehrere Vektor(en), der/die Wildtyp-Gag, Gag, verknüpft mit MCP-3-Aminosäuren 33-109, verknüpft mit sekretorischem Peptid IP-10 MNPSAAVIFCLILLGLSGTQ, und Gag, verknüpft mit β-catenin-Aminosäuren 18-47, codiert/codieren oder
(ii) einen oder mehrere Vektor(en), der/die Wildtyp-Env, Env, verknüpft mit MCP-3-Aminosäuren 33-109, verknüpft mit sekretorischem Peptid IP-10 MNPSAAVIFCLILLGLSGTQ, und Env, verknüpft mit β-catenin-Aminosäuren 18-47, codiert/codieren.

13. Zusammensetzung gemäß einem der vorangehenden Ansprüche, wobei der eine Vektor oder die mehreren Vektoren in Viruspartikeln enthalten sind.

14. Pharmazeutische Zusammensetzung, umfassend einen oder mehrere Vektor(en), wie in einem der Ansprüche 1 bis 12 definiert, und einen pharmazeutisch verträglichen Träger.

15. Pharmazeutische Zusammensetzung gemäß Anspruch 14 zur Verwendung bei der Induzierung von Antikörpern in einem Säuger, wobei die Nucleinsäurekonstrukte in der Zusammensetzung in einer Menge vorliegen, die wirksam ist, um die Antikörper in dem Säuger zu induzieren.

16. Pharmazeutische Zusammensetzung gemäß Anspruch 14 zur Verwendung bei der Induzierung zytotoxischer und/oder Helfer-Inducer-T-Lymphozyten bei einem Säuger, wobei die Nucleinsäurekonstrukte in der Zusammensetzung in einer Menge vorliegen, die wirksam ist, um zytotoxische und/oder Helfer-Inducer-T-Lymphozyten in dem Säuger zu induzieren.

17. Impfstoffzusammensetzung zur Verwendung bei der Induzierung von Immunität bei einem Säuger gegen HIV-Infektion, die eine therapeutisch wirksame Menge eines Vektors oder mehrerer Vektoren, wie in einem der Ansprüche 8 bis 12 definiert, und einen pharmazeutisch verträglichen Träger umfasst.

18. Pharmazeutische Zusammensetzung, die virale Partikel, wie in Anspruch 13 definiert, umfasst.

19. Pharmazeutische Zusammensetzung gemäß Anspruch 14 oder 18 zur Verwendung bei der Stimulierung einer Immunantwort gegen ein Antigen.

20. Zusammensetzung gemäß einem der Ansprüche 1 bis 13 zur Verwendung bei der Impfung eines Säugers durch Induzieren von Antikörpern in dem Säuger, wobei die Vektoren in einer Menge vorliegen, die wirksam ist, um die Antikörper in dem Säuger zu induzieren.

21. Zusammensetzung gemäß einem der Ansprüche 1 bis 13 zur Verwendung bei der Impfung eines Säugers durch Induzieren zytotoxischer und/oder Helfer-Inducer-T-Lymphozyten in dem Säuger, wobei die Vektoren in einer Menge vorliegen, die wirksam ist, um in dem Säuger zytotoxische und/oder Helfer-Inducer-T-Lymphozyten zu induzieren.

22. Kit, umfassend einen Vektor oder mehrere Vektoren, wie in einem der Ansprüche 1 bis 12 definiert.

23. Erster Vektor zur Verwendung in einem Verfahren zur Impfung eines Säugers durch Induzieren von Antikörpern oder zytotoxischen und/oder Helfer-Inducer-Lymphozyten, wobei das Verfahren außerdem eine Verabreichung eines zweiten Vektors umfasst,
wobei einer der Vektoren ein Nucleinsäurekonstrukt exprimiert, das Nucleotidsequenzen enthält, die ein Fusionsprotein codiert, das eine destabilisierende Aminosäuresequenz, die Protein zu dem Ubiquitin-Proteasom-Abbauweg targetiert, kovalent an ein Antigen gebunden umfasst, wobei die Immunogenität des Antigens durch das Vorliegen der destabilisierenden Aminosäuresequenz erhöht ist, und
der andere Vektor ein Nucleinsäurekonstrukt exprimiert, das ein sekretiertes Fusionsprotein codiert, welches eine MCP-3-Chemokin-Aminosäuresequenz kovalent an das Antigen gebunden umfasst, wobei die Immunogenität des Antigens durch das Vorliegen der MCP-3-Chemokin-Aminosäuresequenz erhöht ist.

24. Erster Vektor zur Verwendung in dem Verfahren gemäß Anspruch 23, wobei in dem einen Vektor, der ein Nucleinsäurekonstrukt exprimiert, das Nucleotidsequenzen enthält, die ein Fusionsprotein codieren, das eine destabilisierende Aminosäuresequenz umfasst, die destabilisierende Aminosäuresequenz ausgewählt ist aus der Gruppe, bestehend aus c-Myc-aa2-120, Cyclin-A-aa13-91; Cyclin-B-10-95; Cyclin-B-aa13-91; IkBa-aa20-45; β-Catenin-aa19-44; c-Jun-aa1-67 und c-Mos-aa1-35.

25. Erster Vektor zur Verwendung in dem Verfahren gemäß Anspruch 23 oder 24, wobei der Vektor an die selbe Stelle wie der zweite Vektor verabreicht wird.

26. Erster Vektor zur Verwendung in dem Verfahren gemäß Anspruch 23 oder 24, wobei der Vektor an eine andere Stelle als der zweite Vektor verabreicht wird.

27. Erster Vektor zur Verwendung in dem Verfahren gemäß Anspruch 23 oder 24, wobei der Vektor zur selben Zeit wie der zweite Vektor verabreicht wird.

28. Erster Vektor zur Verwendung in dem Verfahren gemäß Anspruch 23 oder 24, wobei der Vektor zu einer anderen Zeit als der zweite Vektor verabreicht wird.

29. Erster Vektor zur Verwendung in dem Verfahren gemäß Anspruch 23 oder 24, wobei das Antigen ein HIV-Antigen ist.

30. Erster Vektor zur Verwendung in dem Verfahren gemäß Anspruch 23 oder 24, wobei das Antigen HIV-gag ist.

## Revendications

1. Composition comprenant un ou plusieurs vecteur(s) qui exprime(nt) :
i) une construction d'acide nucléique contenant des séquences de nucléotides qui codent une protéine de fusion comprenant une séquence d'acides aminés déstabilisante qui cible une protéine pour la voie de dégradation ubiquitine-protéasome, attachée de manière covalente à un antigène, duquel antigène le caractère immunogène est renforcé par la présence de la séquence d'acides aminés déstabilisante,
ii) et une construction d'acide nucléique codant une protéine de fusion sécrétée qui comprend une séquence d'acides aminés de chimiokine MCP-3 attachée de manière covalente à l'antigène, duquel antigène le caractère immunogène est renforcé par la présence de la séquence d'acides aminés de chimiokine MCP-3.

2. Composition conforme à la revendication 1, dans laquelle la séquence d'acides aminés déstabilisante du composant (i) se trouve dans une séquence d'acides aminés choisie parmi les suivantes : acides aminés n° 2 à 120 de c-Myc, acides aminés n° 13 à 91 de cycline A, acides aminés n° 10 à 95 de cycline B, acides aminés n° 13 à 91 de cycline B, acides aminés n° 20 à 45 de IkBa, acides aminés n° 19 à 44 de β-caténine, acides aminés n° 1 à 67 de c-Jun, et acides aminés n° 1 à 35 de c-Mos.

3. Composition conforme à la revendication 1, dans laquelle la séquence déstabilisante est choisie parmi les suivantes : acides aminés n° 1 à 35 de c-Mos, acides aminés n° 10 à 95 de cycline B, acides aminés n° 19 à 44 de β-caténine, et acides aminés n° 18 à 47 de β-caténine.

4. Composition conforme à la revendication 1, dans laquelle la séquence déstabilisante est choisie parmi les séquences d'acides aminés n° 19 à 44 de β-caténine et n° 18 à 47 de β-caténine.

5. Composition conforme à l'une des revendications précédentes, dans laquelle la séquence d'acides aminés de chimiokine MCP-3 est la séquence des acides aminés n° 33 à 109 ou n° 1 à 109 de MCP-3.

6. Composition conforme à l'une des revendications précédentes, dans laquelle l'antigène est un antigène associé à une maladie.

7. Composition conforme à la revendication 6, dans laquelle l'antigène associé à une maladie est choisi dans l'ensemble formé par un antigène associé à une tumeur, un antigène associé à une maladie auto-immune, un antigène associé à une maladie infectieuse, un antigène viral, un antigène parasitaire et un antigène bactérien.

8. Composition conforme à la revendication 7, dans laquelle l'antigène viral est un antigène de VIH.

9. Composition conforme à la revendication 8, dans laquelle ledit antigène de VIH est choisi dans l'ensemble formé par Gag, Env, Pol, Nef, Vpr, Vpu, Vif, Tat et Rev.

10. Composition conforme à la revendication 6, dans laquelle l'antigène associé à une maladie comprend des fragments antigéniques de Gag-Pol-Tat-Rev-Nef ou Tat-Rev-Env-Nef de VIH reliés ensemble, mais pas obligatoirement dans cet ordre.

11. Composition conforme à la revendication 1, dans laquelle la construction d'acide nucléique codant une protéine de fusion sécrétée est choisie dans l'ensemble formé par les suivantes :
a) une construction comprenant une séquence codant la protéine Gag p37 de VIH, une séquence de chimiokine MCP-3 et une séquence de chimiokine IP-10 ;
b) et une construction comprenant une séquence codant la protéine Gag p39 de VIS, une séquence de chimiokine MCP-3 et une séquence de tête de chimiokine IP-10.

12. Composition conforme à la revendication 1, comprenant :
i) un ou plusieurs vecteurs qui codent une protéine Gag de type sauvage, une protéine Gag raccordée aux acides aminés n° 33 à 109 de MCP-3, eux-mêmes raccordés au peptide sécrétoire d'IP-10 MNPSAAVIFCLILLGLSGTQ, et une protéine Gag raccordée aux acides aminés n° 18 à 47 de β-caténine ;
ii) ou un ou plusieurs vecteurs qui codent une protéine Env de type sauvage, une protéine Env raccordée aux acides aminés n° 33 à 109 de MCP-3, eux-mêmes raccordés au peptide sécrétoire d'IP-10 MNPSAAVIFCLILLGLSGTQ, et une protéine Env raccordée aux acides aminés n° 18 à 47 de β-caténine.

13. Composition conforme à l'une des revendications précédentes, dans laquelle le ou les vecteur(s) est ou sont contenu(s) dans des particules virales.

14. Composition pharmaceutique comprenant un ou plusieurs vecteur(s) de type défini dans l'une des revendications 1 à 12 et un véhicule pharmacologiquement admissible.

15. Composition pharmaceutique conforme à la revendication 14 pour utilisation dans le but d'induire la production d'anticorps chez un mammifère, dans laquelle composition lesdites constructions d'acide nucléique se trouvent présentes en une quantité telle qu'elles induisent efficacement la production desdits anticorps chez ledit mammifère.

16. Composition pharmaceutique conforme à la revendication 14 pour utilisation dans le but d'induire la production de lymphocytes T cytotoxiques et/ou auxiliaires-inducteurs chez un mammifère, dans laquelle composition lesdites constructions d'acide nucléique se trouvent présentes en une quantité telle qu'elles induisent efficacement la production de lymphocytes T cytotoxiques et/ou auxiliaires-inducteurs chez ledit mammifère.

17. Composition de vaccin pour utilisation dans le but d'induire chez un mammifère une immunité contre une infection par VIH, comprenant, en une quantité à effet thérapeutique, un ou plusieurs vecteur(s) de type défini dans l'une des revendications 8 à 12 et un véhicule pharmacologiquement admissible.

18. Composition pharmaceutique comprenant des particules virales de type défini dans la revendication 13.

19. Composition pharmaceutique conforme à la revendication 14 ou 18, pour utilisation dans le but de stimuler une réponse immunitaire contre un antigène.

20. Composition conforme à l'une des revendications 1 à 13, pour utilisation dans le but de vacciner un mammifère en induisant la production d'anticorps chez ce mammifère, dans laquelle lesdits vecteurs se trouvent présents en une quantité telle qu'ils induisent efficacement la production desdits anticorps chez ledit mammifère.

21. Composition conforme à l'une des revendications 1 à 13, pour utilisation dans le but de vacciner un mammifère en induisant la production de lymphocytes T cytotoxiques et/ou auxiliaires-inducteurs chez ce mammifère, dans laquelle lesdits vecteurs se trouvent présents en une quantité telle qu'ils induisent efficacement la production de lymphocytes T cytotoxiques et/ou auxiliaires-inducteurs chez ledit mammifère.

22. Trousse comportant un ou plusieurs vecteur(s) de type défini dans l'une des revendications 1 à 12.

23. Un premier vecteur pour utilisation dans un procédé de vaccination d'un mammifère par induction de la production d'anticorps ou de la production de lymphocytes T cytotoxiques et/ou auxiliaires-inducteurs, ledit procédé comportant en outre l'administration d'un deuxième vecteur, étant entendu que
l'un des vecteurs exprime une construction d'acide nucléique contenant des séquences de nucléotides qui codent une protéine de fusion comprenant une séquence d'acides aminés déstabilisante qui cible une protéine pour la voie de dégradation ubiquitine-protéasome, attachée de manière covalente à un antigène, duquel antigène le caractère immunogène est renforcé par la présence de la séquence d'acides aminés déstabilisante,
et l'autre vecteur exprime une construction d'acide nucléique codant une protéine de fusion sécrétée, comprenant une séquence d'acides aminés de chimiokine MCP-3 attachée de manière covalente à l'antigène, duquel antigène le caractère immunogène est renforcé par la présence de la séquence d'acides aminés de chimiokine MCP-3.

24. Premier vecteur pour utilisation dans le procédé de la revendication 23, dans lequel, pour ledit vecteur exprimant une construction d'acide nucléique contenant des séquences de nucléotides qui codent une protéine de fusion comprenant une séquence d'acides aminés déstabilisante, cette séquence d'acides aminés déstabilisante est choisie parmi les suivantes : acides aminés n° 2 à 120 de c-Myc, acides aminés n° 13 à 91 de cycline A, acides aminés n° 10 à 95 de cycline B, acides aminés n° 13 à 91 de cycline B, acides aminés n° 20 à 45 de IkBa, acides aminés n° 19 à 44 de β-caténine, acides aminés n° 1 à 67 de c-Jun, et acides aminés n° 1 à 35 de c-Mos.

25. Premier vecteur pour utilisation dans le procédé de la revendication 23 ou 24, lequel vecteur est administré au niveau du même site que le deuxième vecteur.

26. Premier vecteur pour utilisation dans le procédé de la revendication 23 ou 24, lequel vecteur est administré au niveau d'un autre site que le deuxième vecteur.

27. Premier vecteur pour utilisation dans le procédé de la revendication 23 ou 24, lequel vecteur est administré au même moment que le deuxième vecteur.

28. Premier vecteur pour utilisation dans le procédé de la revendication 23 ou 24, lequel vecteur est administré à un autre moment que le deuxième vecteur.

29. Premier vecteur pour utilisation dans le procédé de la revendication 23 ou 24, pour lequel l'antigène est un antigène de VIH.

30. Premier vecteur pour utilisation dans le procédé de la revendication 23 ou 24, pour lequel l'antigène est une protéine Gag de VIH.
